(19) Europäisches Patentamt
European Patent Office
Office européen des brevets

(11) Numéro de publication : **0 394 126 B1**

(12) # FASCICULE DE BREVET EUROPEEN

(45) Date de publication du fascicule du brevet : **20.07.94 Bulletin 94/29**

(51) Int. Cl.$^5$ : **C07D 207/335,** C07D 213/38, C07C 323/60, A61K 49/02

(21) Numéro de dépôt : **90401046.9**

(22) Date de dépôt : **18.04.90**

(54) **Composés et complexes utiles notamment en imagerie médicale.**

(30) Priorité : **19.04.89 FR 8905215**
**01.12.89 FR 8915896**

(43) Date de publication de la demande : **24.10.90 Bulletin 90/43**

(45) Mention de la délivrance du brevet : **20.07.94 Bulletin 94/29**

(84) Etats contractants désignés : **AT BE CH DE DK ES FR GB GR IT LI LU NL SE**

(56) Documents cités :
**EP-A- 0 123 504**
**EP-A- 0 163 119**
**EP-A- 0 200 492**

(73) Titulaire : **MEDGENIX DIAGNOSTICS**
**Zoning Industriel**
**Rue de l'Espérance**
**B-6220 Fleurus (BE)**

(72) Inventeur : **Thornback, John R.**
**Avenue de l'Aulne 37**
**Uccle, B-1180 Bruxelles (BE)**
Inventeur : **Deblaton, Marcel**
**13, rue St. Sauveur**
**B-5854 Meux (BE)**
Inventeur : **Morgan, Gillian F.**
**Avenue de l'Aulne 37**
**Uccle, B-1180 Bruxelles (BE)**

(74) Mandataire : **Warcoin, Jacques et al**
**Cabinet Régimbeau**
**26, avenue Kléber**
**F-75116 Paris (FR)**

EP 0 394 126 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente demande de brevet concerne des ligands susceptibles de complexer des métaux. Lorsque ces métaux sont radioactifs, ces complexes sont utiles notamment en imagerie médicale ou en radiothérapie ciblée selon la nature du radioisotope.

La présente invention concerne également des kits contenant lesdits ligands, lesdits métaux et des réactifs permettant de conduire à la formation d'un complexe de coordination entre les ligands et les métaux.

Plus particulièrement, la présente invention concerne des complexes entre ces ligands et du technetium $^{99m}$Tc utiles comme agents de diagnostic, en particulier pour l'imagerie du cerveau, du coeur et l'imagerie sanguine.

Les conditions requises pour un agent d'imagerie du cerveau sont tout d'abord sa faculté à traverser la barrière hémato-encéphalique, se fixer et s'accumuler à grande concentration dans un temps assez court dans le cerveau et y rester pendant 1 durée nécessaire requise pour effectuer l'analyse. Ceci nécessite que le complexe subisse des modifications chimiques ou biochimiques in vivo. On a suggéré par exemple un mécanisme de déplacement de pH qui prend en compte la différence de pH entre le sang et le milieu intracellulaire. Ainsi, si une amine a une valeur de pKa proche du pH du cerveau, une fois à l'intérieur de celui-ci le complexe amine neutre sera protoné. Le complexe ainsi chargé est piégé dans le cerveau dans la mesure où il n'est pas capable d'en sortir par un mécanisme inverse compte tenu du pKa de l'amine. Ce complexe peut également subir une modification chimique. C'est le cas avec le HM-PAO où le complexe lipophile se transforme dans le temps en un complexe plus hydrophile qui ne pénètre pas dans le cerveau. La nature exacte de ce second complexe et le mécanisme qui entre en jeu n'ont pas encore été élucidés. Il est encore possible que l'hydrolyse enzymatique d'un groupe actif se produise comme on l'observe avec le "N,N'-1,2-éthylenediylbis-L-cystéine diéthyl ester" ($^{99m}$Tc ECD) où l'ester est hydrolysé en stéréoisomère L,L et non son isomère D,D d'où il résulte que le temps de rétention dans le cerveau est différent entre les deux isomères avec un temps demi vie pour le premier supérieur à 1 440 minutes et inférieur à 30minutes pour le dernier.

Des complexes de ce type ont été décrits dans EP-A-0163119, EP-A-0123504, EP-A-0-200492.

Quel que soit le mécanisme qui entre en jeu, un complexe idéal dans une optique d'imagerie du cerveau devra avoir une bonne stabilité in vitro, une fixation rapide dans le cerveau, et une faible stabilité in vivo c'est-à-dire une faculté de modification ou de transformation in vivo.

Contrairement à l'opinion établie en ce qui concerne les agents d'imagerie du coeur, il apparaît qu'ils ne doivent pas nécessairement consister en un complexe cationique, mais que plus vraisemblablement c'est leur propriété lipophile qui est déterminante.

La lipophilicité des complexes leur confére la faculté de marquer des cellules du sang telles que les macrophages et de permettre ainsi la détection d'inflammation et d'infections.

L'isotope radioactif de choix pour l'imagerie médicale est le $^{99m}$Tc. Actuellement, dans les applications d'imagerie du cerveau, du coeur et des sites inflammatoires, des "kits froids" marquables au $^{99m}$Tc ne sont pas encore commercialement disponibles (MIBI : imagerie du coeur) ou présmentent certains inconvénients (HMPAO : imagerie du cerveau et des sites inflammatoires). Le $^{99m}$Tc est généralement disponible sous forme de pertechnétate ($TcO^-_4$). Les kits froids comprennent une composition lyophilisée de composants non-radioactifs qui, après reconstitution au moyen d'une solution de pertechnétate $^{99m}TcO^-_4$, donne le complexe désiré. Idéalement, la stabilité du complexe après reconstitution doit être suffisamment bonne de sorte que le kit puisse être utilisé pendant plusieurs heures. Un problème fréquent rencontré avec les complexes actuels est que le complexe de $^{99m}$Tc ne garantit pas une stabilité dans le temps suffisante.

En outre, les ligands proposés jusqu'à maintenant présentent en général des structures chimiques compliquées entrainant des méthodes de préparation coûteuses.

Dans le but de proposer des agents de diagnostic éventuellement polyvalents permettant l'imagerie notamment du cerveau, du coeur et de cellules sanguines telles que les macrophages, on a recherché selon la présente invention de nouveaux ligands lipophiles et neutre présentant les meilleures spécifications requises à cet effet et qui pallient aux inconvénients précités.

La présente invention a en effet pour objet de nouveaux composés répondant à la formule générale suivante :

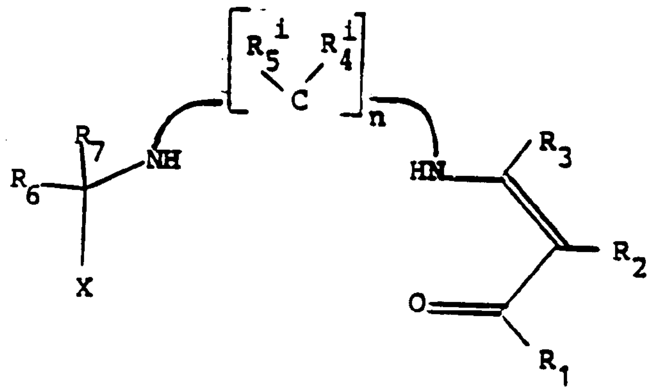

(I)

dans laquelle

$R_1$ et $R_3$ représentent indépendamment l'un de l'autre H, un groupe alkyle, alkényle, cycloalkyle, aryle, arylalkyle, un alkylaryle, un hétérocycle, groupes éventuellement substitués, notamment par un ou des groupes hydroxy, alcoxy ou halogène.

n représente un nombre entier de 1 à 5

i prend respectivement les valeurs de 1 à n pour les n maillons

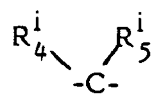

successifs.

$R_2$, $R_4^i$ et $R_5^i$ représentent indépendamment l'un de l'autre H, un groupe alkyle, alkényle, aryle, alcoxy, hydroxyalkyle, alcoxyalkyle, amide, acyle ou carboxyalkvle, ou un sel, ou un ester alkylique de ce dernier, ou $R_4^i$ et $R_5^i$ forment ensemble un groupe oxo

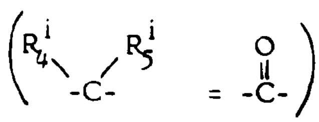

$R_6$, $R_7$ représentent H,

X représente un hétérocycle de 5 ou 6 chainons contenant au moins un atome d'azote.

Dans la présente demande, on entend par acyle, alkyle, hydroxy- carboxy- ou alcoxyalkyle et alcoxy des chaînes droites ou ramifiées de 1 à 10 atomes de carbone.

Le terme alkényle se référe également à des chaînes droites ou ramifiées, de 2 à 10 atomes de carbone.

Le terme aryle se référe au groupe phényle.

Le terme cycloalkyle se référe à des cycles ayant 5, 6 ou 7 atomes de carbones.

On entend par hétérocycle à 5 ou 6 chaînons contenant au moins un atome un azote, un des composés représentés par la formule II ou un de leurs dérivés déhydro :

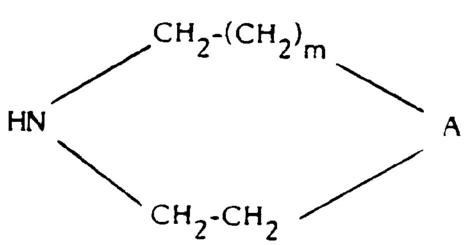

(II)

dans laquelle

m est 0 ou 1 et

A est O, N-$R_{11}$ ou CH-$R_{11}$ où $R_{11}$ représente H, alkyle, aryle, éventuellement substitués.

De telles hétérocycles sont par exemple des groupes aromatiques tels qu'un groupe pyrrolyle, imidazolyle, oxazolyle, pyrazolyle, pyridinyle, pyrimidinyle.

Ils peuvent être rattachés au ligand de formule I par un carbone insaturé adjacent à N ou A.

On peut citer plus particulièrement comme composé le composé de formule I selon l'invention, les composés pour lesquels n = 2 ou 3, et aussi ceux pour lesquels

. X =

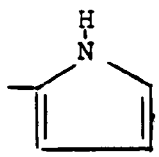
,
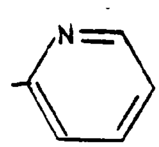

On citera encore plus particulièrement les composés pour lesquels d'une manière générale $R_4^i = R_5^i$ = H avec i = 1 à n ou ceux pour lesquels $R_4^i = R_5^i$ = H quand i = 1 à n-1 et

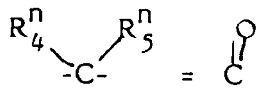

Enfin on peut citer les composés pour lesquels $R_1$ et $R_3$ représentent indépendamment l'un de l'autre H, $CH_3$, $CF_3$, $C(CH_3)_3$, $C_2H_5$.

Certains composés selon l'invention répondent à la formule III:

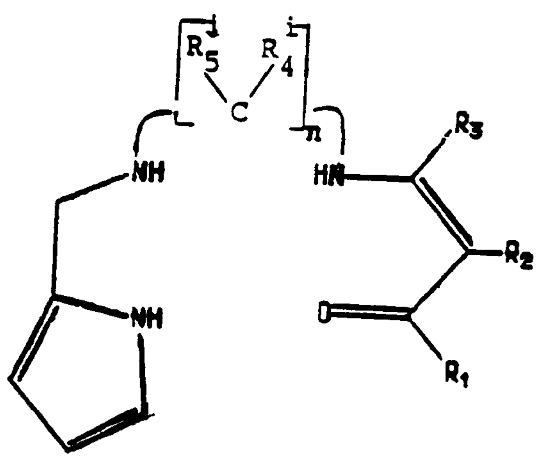

(III)

dans laquelle $R_1$, $R_2$, $R_3$, $R^i_4$ $R^i_5$ ont les significations données précédemment.

On peut citer en particulier comme ligands de formule III ceux pour lesquels $R_4^i = R_5^i$ = H.

On donne comme exemple de ligands de formule III particulièrement intéressants les composés où

1) $R_1$, $R_3$ = $CH_3$ ou $C_2H_5$, n = 2 et $R_2 = R_4^1 = R_4^2 = R_5^1 = R_5^2$ = H ;

2) $R_1$ = $CH_3$, $R_3$ = $CF_3$, n = 2 et $R_2 = R_4^1 = R_4^2 = R_5^1 = R_5^2$ = H,

3) $R_1$ = $CF_3$, $R_3$ = $CH_3$, n = 1 et $R_2 = R_4^1 = R_4^2 = R_5^1 = R_5^2$ = H

4) $R_1 = R_3 = C(CH_3)_3$, n = 2 et $R_2 = R_4^1 = R_4^2 = R_5^1 = R_5^2$ = H ;

5) $R_1 = R_2 = R_3 = CH_3$ et n = 2, $R_4^1 = R_4^2 = R_5^1 = R_5^2$ = H,

6) $R_1 = R_3 = CH_3$, n = 3 et $R_2 = R_4^1 = R_4^2 = R_4^3 = R_5^1 = R_5^2 = R_5^3$ = H.

D'autres composés selon l'invention répondent à la formule IV :

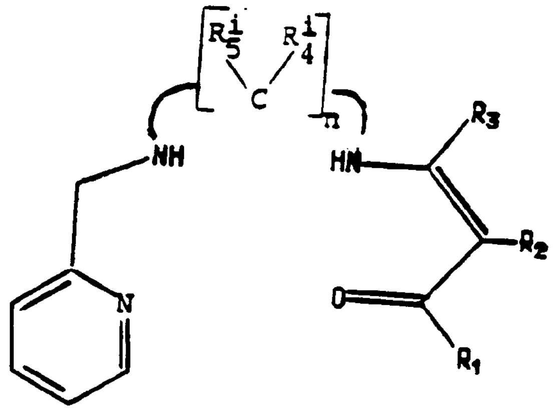

(IV)

dans laquelle $R_1$, $R_2$, $R_3$, $R_4^i$, $R_5^i$ ont les significations données précédemment dans la formule générale de I.

On peut citer comme composé plus particulièrement intéressant de formule IV, celui où n = 2 et $R_2 = R_4^i = R_5^i = H$.

Parmi ces derniers, on donne l'exemple où n = 2, $R_1 = R_3 = CH_3$ et $R_2 = H$.

D'autres composés selon l'invention répondent à la formule V :

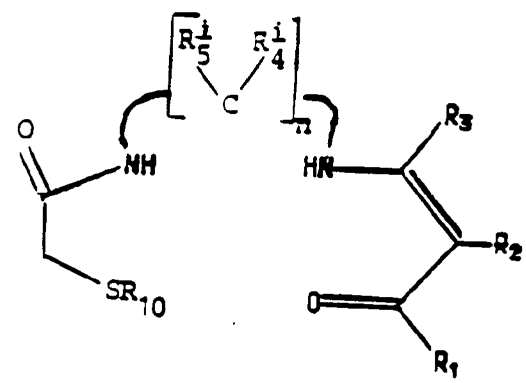

(V)

dans laquelle $R_1$, $R_2$, $R_3$, $R_4^i$, $R_5^i$, $SR_{10}$ ont les significations données précédemment pour la définition de la formule générale I.

On peut citer plus particulièrement les composés de formule V où $R_{10}$ = trityl, n = 2 ou 3 et $R_4^i = R_5^i = H$.

Parmi ces derniers, on donne un exemple avec $R_1 = R_3 = CH_3$ et $R_2 = H$.

D'autres composés selon l'invention répondent à la formule générale VI

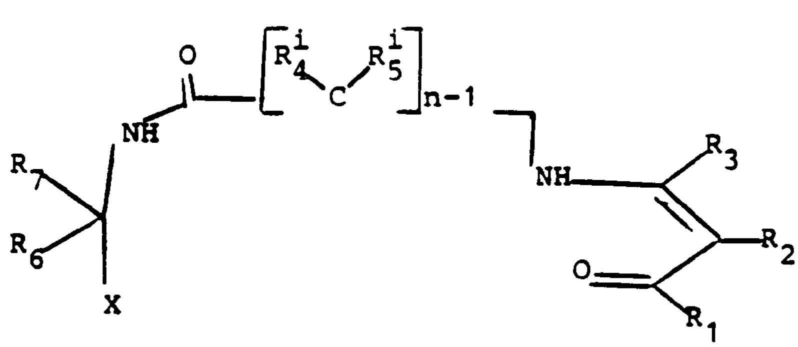

VI)

dans laquelle $R_1$ à $R_7$ ont les significations données précédemment.

Parmi les composés de formule VI, on peut citer ceux pour lesquels $R_6 = R_7 = H$, et X représente un hétérocycle. On citera encore ceux pour lesquels $R_4^i = R_5^i = H$.

On cite comme exemple le composé de formule VI dans lesquels n = 2, $R_2 = R_4^i = R_5^i = R_6 = R_7 = H$, $R_1 = R_3 = CH_3$ ou Et, et X est un groupe pyrrolyle.

La présente invention a également pour objet un procédé de préparation des composés selon l'invention.

Ces composés peuvent en effet être préparés en faisant réagir un hétérocycle X substitué par un groupe formyle de formule

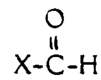

avec une fonction amine d'un alkylène diamique de formule $NH_2-(CR_4^iR_5^i)_n-NH_2$ dans de l'acétonitrile en présence de $NaBH_4$.

Enfin, lorsque

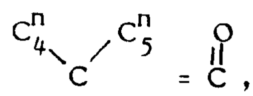

on fait réagir une amine de formule $X(CR_6R_7)\ NH_2$ avec l'amino-acide HOOC $(CR_4^i\ R_5^i)_{n-1}-NH_2$ en activant la fonction acide avec un groupe activateur conventionnel dans la chimie peptidique et en protégeant la fonction $NH_2$ par un groupe protecteur également conventionnel.

On obtient dans tous les cas un composé de formule VII

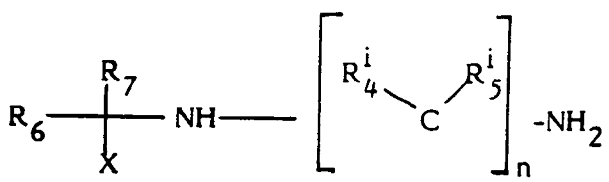

(VII)

Ensuite, on fait réagir le produit obtenu de formule VII avec une dione de formule VIII :

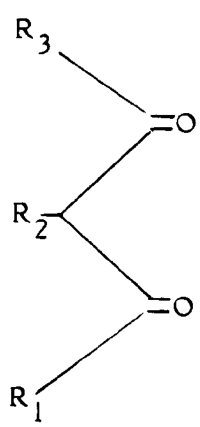

(VIII)

pour obtenir le composé de formule (I) selon l'invention.

Les composés décrits dans l'invention ont une utilité comme ligands pouvant former des complexes avec des métaux. Ces complexes, lorsqu'ils sont injectés in vivo, peuvent présenter une affinité pour les cellules cérébrales ou myocardiques. Ils peuvent également être utilisés pour le marquage des globules blancs, les globules blancs marqués pouvant être injectés in vivo pour la détection de sites inflammatoires. Selon l'isotope utilisé pour le marquage, les composés pourront être utilisés comme agents d'imagerie ou comme agents de pilotage en radiothérapie ciblée.

Les composés selon l'invention peuvent se trouver sous toutes les formes acide ou basique.

Les composés décrits dans l'invention peuvent être utilisés soit directement, soit couplés à une protéine

transporteuse telle qu'un anticorps monoclonal. Leur rôle se réduit alors à coupler un métal à la protéine.

La présente invention a donc également pour objet des complexes de coordination d'un composé décrits dans l'invention avec un ion métallique radioactif ou paramagnétique.

On peut citer notamment les radioisotopes $^{111}In$, $^{113m}In$, $^{67}Ga$, $^{68}Ga$, $^{157}Gd$, $^{201}Ti$, $^{117m}Sn$, $^{64}Cu$, $^{186}Re$, $^{188}Re$, $^{90}Y$, $^{212}Bi$, $^{99}Tc$, $^{99m}Tc$, $^{97}Ru$, $^{51}Cr$, $^{57}Co$, $^{191}Os$.

Les composés selon l'invention peuvent être marqués par un isotope radioactif d'halogène substitué sur la molécule constituant ledit composé, notamment $^{123}I$, $^{125}I$, $^{131}I$.

Le choix de l'isotope radioactif dépend de l'utilisation à laquelle est destinée le ligand.

Pour une utilisation à titre d'agent d'imagerie on utilisera par exemple les ligands marqués avec le $^{99m}Tc$, ou encore $^{111}In$, $^{113m}In$, $^{67}Ga$, $^{68}Ga$, $^{157}Gd$, $^{123}I$, $^{131}I$, $^{201}Ti$, $^{117m}Sn$.

A des fins de radiothérapie ciblée les ligands seront par exemple marqués avec $^{125}I$, $^{131}I$, $^{64}Cu$, $^{186}Re$, $^{90}Y$, $^{212}Bi$.

La présente invention a donc aussi pour objet des kits comprenant les ligands selon l'invention et des réactifs permettant de conduire à la formation de complexes de coordination entre lesdits ligands et les métaux cités plus haut.

Le ligand peut se trouver en solution hydro-alcoolique ou sous forme lyophilisée.

Lesdits réactifs sont en général des agents réducteurs, et des agents stabilisants comme l'acide paraaminobenzoïque ou une base dont certains exemples sont fournis dans la demande de brevet principale.

Selon un mode préférentiel d'utilisation des composés selon l'invention, ceux-ci sont utilisés pour complexer du $^{99m}Tc$ à des fins d'imagerie médicale du cerveau, du coeur, des infections ou le marquage des lymphocytes pour l'imagerie des sites inflammatoires.

Les kits comportent alors le ligand et comme agent réducteur de préférence un sel d'étain tel que du chlorure stanneux. Le composé métallique utilisé pour reconstituer le kit est le pertechnétate $^{99m}TCO_4^-$. Le technetium se trouve vraisemblablement sous forme d'oxyde Tc = 0 dans les complexes formés.

D'autres caractéristiques et avantages de la présente invention apparaîtront à la lumière des exemples de réalisation de l'invention qui vont suivre.

La figure 1 représente un graphique de clairance du complexe $^{99m}Tc$ N[-2(1H pyrolylméthyl)] N'[-4-(pentene-3-one-2)] éthylene 1,2 diamine (MRP20) (pourcentage de doses injectées par gramme de tissus).

Les figures 2 et 4 représentent un graphique de biodistribution des complexes $^{99m}Tc$ de MRP30 et MRP26 respectivement.

La figure 3 représente un graphique de clairance du complexe MRP27.

**EXEMPLE 1: SYNTHESE DU LIGAND N[-2(-1H-pyrrolylméthyl)] N'[-4-(pentène-3-one-2)] éthylène 1,2 diamine (ci-après MRP20)**

Composé de formule I dans lequel $R_1 = R_3 = CH_3$ et $R_2 = R_4^1 = R_4^2 = R_5^1 = R_5^2 = H$ et n = 2

1) Synthèse du N[-2(-1H pyrrolylméthyl)] éthylène 1, 2 diamine

Sous une atmosphère d'argon, on ajoute une solution de 0,1 mol (9,5 g) de pyrrol-2-carboxaldéhyde dans 50 ml d'acétonitrile sec à une solution de 0,6 mol (40 ml) d'éthylène 1,2 diamine dans 80 ml d'acétonitrile sec contenant 2 g de tamis moléculaire. Le mélange est laissé sous agitation à température ambiante pendant une nuit. Le solvant est évaporé sous vide et le résidu est redissout dans 100 ml de méthanol. La solution obtenue est placée dans un bain de glace et, sous forte agitation, 0,1 mol de borohydrure de sodium y est ajouté par petites fractions. Le mélange réactionnel est ensuite laissé sous agitation pendant deux heures.

Un maximum de méthanol est évaporé sous vide et l'huile obtenue est resolubilisée dans 100 ml d'eau. Sous forte agitation, cette solution est placée dans un bain de glace et additionnée de 20 g d'hydroxyle de potassium. Après l'extraction du mélange par 5 fois 50 ml de dichlorométhane, les phases organiques sont rassemblées, séchées sur du carbonate de potassium anhydre, filtrées puis évaporées pour obtenir un résidu huileux, qui est ensuite placé dans un bain d'eau à 60°C et tiré sous vide. On obtient finalement 14 g d'une huile visqueuse jaune (rendement brut proche de 100 %). Un contrôle en CCM sur silice avec comme éluant un mélange de 5 % d'ammoniaque dans du méthanol indique la présence d'une impureté principale plus polaire. Celle-ci est éliminée par précipitation sélective de son chlorhydrate dans l'éthanol. On obtient finalement 10 g (rendement de 70 %) d'une huile jaune de pureté satisfaisante pour les étapes suivantes.

2) Synthèse du N[-2(-1H-pyrrolylméthyl)] N'[-4-(pentène-3-one-2)] éthylène 1,2 diamine

A une solution de 10 mmol (1,4 g) de 1) dans 10 ml d'acétonitrile, on ajoute 12 mmol (1,2 ml) de 2,4-pentanedione. Le mélange est laissé sous azote pendant trois heures à température ambiante, puis le solvant est évaporé sous vide. L'huile résiduelle est alors réextraite dans un mélange d'eau et de dichlorométhane. Les phases organiques séchées, filtrées puis évaporées fournissent une huile jaune qui est purifiée sur une colonne de silice avec comme éluant un mélange de 0,5 % de diéthylamine dans l'acétate d'éthyle. Le produit final obtenu est une huile jaune pâle qui se resolidifie après réfrigération. La recristallisation dans l'éther diisopropylique fournit 1,5 g de beaux cristaux en forme d'aiguilles (rendement de 66 %).

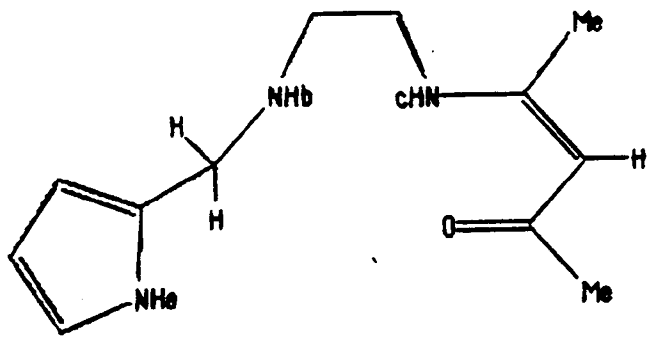

Données analytiques :

| | |
|---|---|
| RMN : | déplacements en ppm 11,2 (s,br) NHc ; 9,6 (s,br) NHa ; 6,75 (qu) 6,08 (qu) 5,98 (tr) pyrolle ; 5,0 (s) CH ; 3,8 (s) $CH_2$ ; 3,29 (qu), 2,83 (tr) $CH_2$-$CH_2$ : 1,9 (s), 2,05 (s) Me, Me ; 1,6 (s, br) NHb. |
| Infrarouge : | N-H, 3174 cm-1, C=O, 1598 cm-1, d'autres bandes sont détectées à 3090, 2972, 2852, 1544, 1466, 1432, 1376, 1357, 1340, 1308, 1280, 1251, 1130, 1097, 1028, 998. |
| Spectrométrie de masse : | ion correspondant à M/e 221,2 : -Me 206,1 : -C=O 178,1 : pics à 127, 113, 98, 95, 84, 80 représentant divers stades de la fragmentation. |

**EXEMPLE 2 : SYNTHESE DU LIGAND N[-2(-1H-pyrrolylmethyl)] N'[-4-(-5-trifluoropentène-3-one-2)] éthylène 1,2 diamine**

Composé de formule I dans laquelle $R_1$ = CF3, $R_3 = CH_3$, $R_2 = R_4^1 = R_4^2 = R_5^1 = R_5^2$ = H et n = 2

A une solution de 10 mmol (1,4 g) du produit obtenu dans l'exemple 1, 1), dans 15 ml de dichlorométhane, on ajoute 1 g de tamis moléculaire et on place le tout dans un bain de glace. On y ajoute alors, et sous agitation, 12 mmol (1,4 ml) de 1,1,1 trifluoro-2,4-pentanedione. le mélange est laissé sous argon pendant une nuit. Après un lavage à l'eau, la phase dichlorométhane est séchée puis évaporée pour fournir une huile jaune. La purification du produit est effectuée par chromatographie sur silice avec un mélange de 0,5 % de diéthylamine dans de l'acétate d'éthyle comme éluant. On obtient une huile jaune pâle qui se resolidifie à froid. Le produit est ensuite recristallisé dans un mélange de 5 % d'éther diisopropylique dans l'hexane. On obtient 0,9 g de cristaux blancs en forme de fines aiguilles (rendement de 30 %).

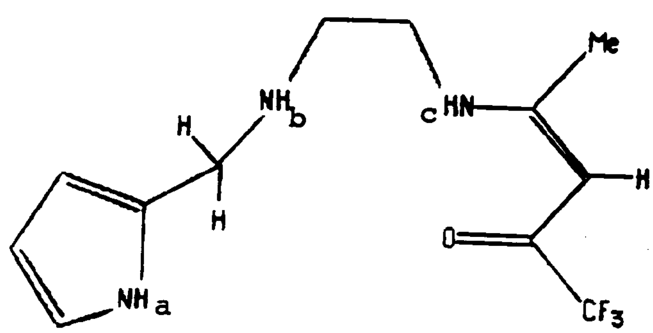

Données analytiques :

| | |
|---|---|
| RMN : | déplacements en ppm 10,9 (s, br) NHc ; 9,4 (s, br) NHa ; 6,75 (qu), 6,08 (qu), 5,98 (tr) pyrolle ; 5,35 (s) CH ; 3,85 (s) $CH_2$ ; 3,38 (qu), 2,9 (tr) $CH_2$-$CH_2$ ; 2,08 (s) Me ; 1,6 (s, br) NHb. |
| Infrarouge : | N-H 3365/3350 cm-1, C=O 1554 cm-1. D'autres bandes ont été observées à 2863, 1605, 1372, 1252, 1125, 1021 cm-1. |
| Spectroscopie de masse : | l'ion moléculaire m/e 275 est accompagné d'un fragment à 255 (perte de HF). Le diagramme de fragmentation met bien en évidence la présence du noyau pyrrole et du squelette éthylène diamine, tandis que les spectres de collision indiquent des pertes supplémentaires de R. |

## EXEMPLE 3

En suivant le mode opératoire des exemples 1 et 2, on a préparé les composés de formules III (X = pyrollyle) suivants :

1) Composé MRP22

$R_1 = R_3 = C(CH_3)_3$ n = 2 $R_4^1 = R_4^2 = R_5^1 = R_5^2 = R_2 = H$

Données spectroscopiques

RMN : déplacements en ppm

10,7 (s, b1) NH (c)
10,2 (s, br) NH (a)
6,77 (qu)
6,09 (qu)
5,99 (tr)
5,35 (5) } pyrrolyle

3,90 (s)
3,52 (qu)
2,90 (tr) } $CH_2$
1,5 (s, br) NH (b)
1,32 (s)
1,2 (s) } $CH_3$

Masse : E1 70 ev
$M^+$ M/Z 305 amu
fragments 248, 198, 196, 184, 169, 140 (BP), 80

| CHN : | Théorique | Trouvé |
|---|---|---|
| C | 71,11 | 70,77 |
| H | 10,23 | 10,40 |
| N | 13,78 | 13,76 |

2) Composé MRP30

$R_1 = R_3 =$ Me n = 3 $R_4^1 = R_4^2 = R_4^3 = H$

$R_5{}^1 = R_5^2 = R_5^3 = R_2 = H$

Données spectroscopiques :

RMN : déplacements en ppm

11,08 NH (c)
10,05 NH (a)

| | |
|---|---|
| 6,75 (qu) | pyrrolyle |
| 6,99 (qu) | |
| 5,99 (br) | |
| 4,97 (s) | |

| | |
|---|---|
| 3,78 (s) | $CH_2$ |
| 3,35 (qu) | |
| 2,80 (tr) | |

| | |
|---|---|
| 2,03 | $CH_3$ |
| 1,91 | |

1,75 (quadr) $CH_2$
1,6 (br) NH (b)

Masse : EI 70 ev
$M^+$ M/Z = 235 (BP)
fragments : 192, 157, 155, 126, 113, 98, 80

| CHN : | Théorique | Trouvé |
|---|---|---|
| C | 66,88 | 67,64 |
| H | 8,94 | 8,96 |
| N | 17,87 | 17,71 |

3) Composé MRP21

$R_1$ = Me $R_3$ = $CF_3$ n = 3 $R_4^1 = R_4^2 = R_4^3$ = H

$R_5^1 = R_5^2 = R_5^3 = H = R_2$

Données spectroscopiques :

RMN : déplacements en ppm

10,9 (S, br) NH (c)
9,4 (S, br) NH (a)
6,75 (qu)
6,08 (qu) pyrrolyle
5,98 (tr)
5,35 (s) CH
3,85 (s)
3,38 (qu) $CH_2$
2,9
2,08 (s) $CH_3$
1,6 (s, br) NH (b)

Masse : EI 70 ev
$M^+$ - M/Z 275 amu.
fragments M/Z 255, 166, 127, 109, 80 (BP)

| CHN : | Théorique | Trouvé |
|---|---|---|
| | C 70,77 | 71,11 |
| | H 10,23 | 10,40 |
| | N 13,76 | 13,78 |

4) Composé MRP23
$R_1 = R_2 = R_3 = Me$ n = 2 $R_4^1 = R_4^2 = R_5^1 = R_5^2 = H = R_2$
Données spectroscopiques :
RMN : déplacements en ppm (mélange d'isomères)

12,15 (bv) NH c
9,85 (bv) NH a
6,90
6,10
6,05 pyrrolyle
6,10

---

4,26 (s)
3,94 (s)
3,60 (tr) $CH_2$
3,31 (qu)
3,18 (tr)
2,85 (tr)

---

2,18 (s)
2,04 (s) $CH_3$
1,94 (s)
1,82 (s)
1,70 (br) NH

Masse : $M^+$ M/Z 235
fragments 221, 163, 127, 112, 110, 80 (BP)

5) Composé MRP27

$R_1 = R_2$ = Ethyl  n = 2 $R_2 = R_4^1 = R_4^2 = R_5^1 = R_5^2 = R_3$ = H

Données spectroscopiques :

RMN : déplacements en ppm

11,4 (br) NH (c)
9,9 (br) NH (a)
6,75 (qu)
6,10 (qu) } CH pyrrolyle
6,97 (tr)
5,00 (s)
3,85 (s)
3,32 (qu) } $CH_2$
3,85 (tr)

---

2,31 (qu)
2,19 (qu)
1,5 (br) NH b
1,15 (quadr) $CH_3$

Masse : EI 70 ev
$M^+$ M/Z 249
fragments 141, 128, 112 (BP), 80.

**EXEMPLE 4: Synthèse du N-2(pyridinylméthyl) N'-4-(pentene-3-one-2) éthylène diamine (ci-après dénommé MRP-50)**

Ce composé a pour formule

Ce

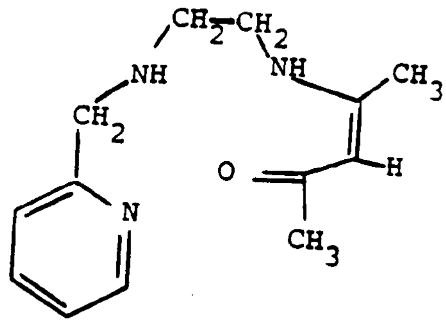

1. Synthèse du N(-2-pyridinylméthyl) éthylène diamine

1.1.

- Sous azote, à un mélange de 8 ml d'éthylènediamine (120 mM) dans 20 ml d'acétonitrile, on ajoute 1 gr de tamis moléculaire, puis très lentement une solution de 1,9 ml (20 mM) de pyridine 2 aldéhyde dans 8 ml acétonitrile.
- On laisse sous agitation la nuit à température ambiante.

1.2.

- On évapore le maximum de solvant.
- On ajoute 20 ml de méthanol et l'on place le ballon de réaction dans un bain de glace.
- On ajoute alors 0,8 gr (20 mM) de borohydrure de sodium.
- On laisse le milieu réactionnel sous agitation à température ordinaire pendant 2 heures.

1.3.

- On évapore le méthanol et l'on ajoute au résidu 20 ml d'eau.
- La solution est placée dans un bain de glace. On y ajoute 4 gr de KOH.
- On extrait par $CH_2Cl_2$. Les phases organiques sont rassemblées, séchées sur $K_2CO_3$ anhydre.

1.4.

- Après évaporation, on obtient 3,1 gr d'une huile rougeatre (rdt brut : environ 100 %) TLC $SiO_2$ MEOH/4% $NH_4OH$.

2. Synthèse de N-2(pyridinylméthyl) N'-4-(pentene-3-one-2) éthylène diamine

2.1

- Les 3,1 gr de 1 brut (20 mM) sont redissous dans 20 ml $CH_3CH$.
- On ajoute 24 mM (2,4 ml) d'acétyl acétone.
- On laisse réagir sous agitation avec environ 1 gr de tamis moléculaire à température ambiante pendant une nuit.

2.2.

- On évapore le maximum de solvant.
- On verse 100 ml d'eau et l'on extrait au $CH_2Cl_2$.
- les phases organiques sont séchées, filtrées puis évaporées, on obtient 2,8 gr huile rouge brun. TLC $SiO_2$/méthanol indique la présence de plusieurs impuretés colorées.

2.3 Purification

- $SiO_2$ éluant acétate d'éthyle/0,5 % DEA
- Le produit se décompose en partie dans ce système.
- On obtient finalement 0,6 gr d'une huile jaune rouge qui est monotache en TLC/$SiO_2$ méthanol.

| | | | | | |
|---|---|---|---|---|---|
| Spectromètrie de masse : MT | M/Z 233 | 4 % | M/Z 121 | 100 % |
| fragm. | M/Z 176 | 3 % | M/Z 109 | 20 % |
| | M/Z 147 | 12 % | M/Z 92 | 27 % |

RMN 1,92 S Me
2,01 S Me
2,82 tr $CH_2$-$CH_2$
3,39 qu $CH_2$-$CH_2$
3,93 S $CH_2$

**<u>EXEMPLE 5: Synthèse du N-[2-(N-(4-) pentene-3-one-2) aminoéthyl]-2-(triphenylméthylthio) acétamide (ci-après MRP-40)</u>**

Le composé répond à la formule suivante :

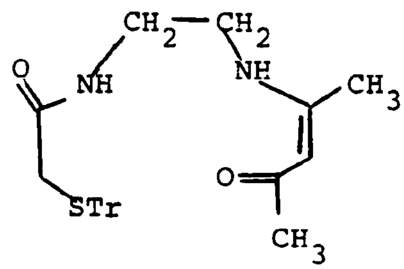

1. <u>Synthèse du N-(2-aminoéthyl)-2-(triphénylméthylthio) acétamide</u>

Le composé a pour formule :

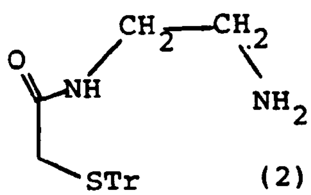

(2)

Le schéma réactionnel est le suivant :

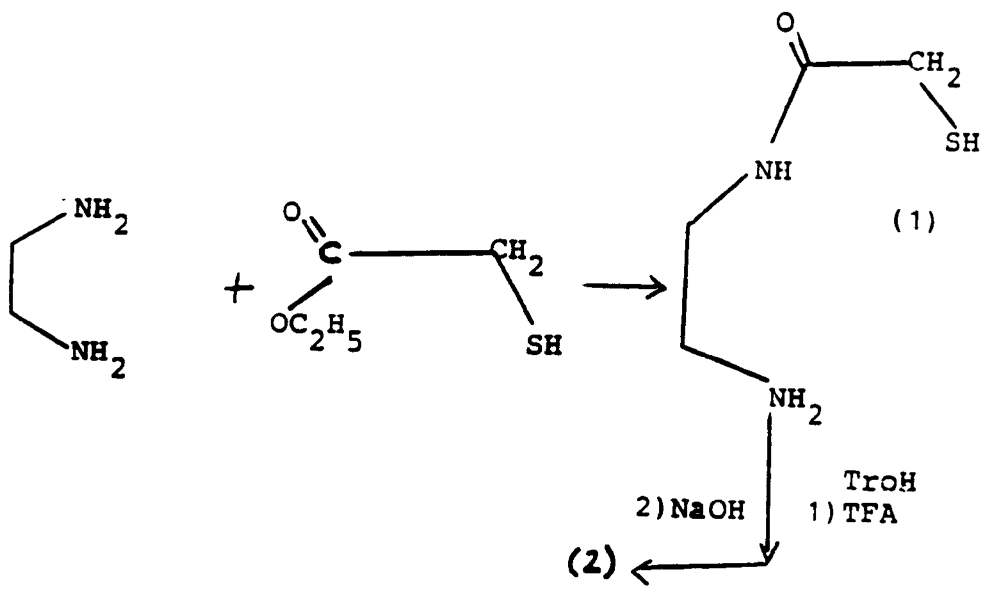


1.1 <u>Synthèse du N-(2-aminoéthyl)-2 thioacétamide (1)</u>

1.1.1.

- A une solution de 10 ml d'éthylènediamine (0,15 M) dans 50 ml d'éthanol absolu, maintenue sous azote sec et à une température inférieure à 10°C (bain de glace), on ajoute lentement une solution de 33 ml (0,30 M) de thioacétate d'éthyle dans 27 ml d'éthanol absolu.

On laisse réagir la nuit à température ambiante.

1.1.2.

- Le précipité blanc est filtré rapidement, lavé à l'alcool puis à l'éther puis séché sous azote.
- On obtient 17,6 g (rendement = 88 %) d'un solide blanc qui est rapidement engagé dans l'étape

suivante.

1.2 Synthèse du N-(2-aminoéthyl)-2-(triphényl méthylthio) acétamide (2)

1.2.1.

- A une solution de 13,4 gr de 1-(0,1 M) dans 100 ml d'acide trifluoroacétique. On ajoute 28,25 gr (0,11 M) de triphényl méthanol.
- La solution brune obtenue est agitée pendant 30 secondes puis évaporée sous vide pour donner une huile brun foncé.
- Celle-ci, triturée avec 500 ml d'éther, nous donne un précipité blanc qui est filtré, lavé à l'éther et séché sous vide.
- On obtient 51 gr d'une poudre blanche (rendement = 80 %).

1.2.2.

- 10 gr de 2 (20 mM) sont partitionnés entre 30 ml de NaOH 1M et 100 ml d'acétate d'éthyle.
- La phase organique est lavée à l'eau puis à l'eau + sel, et séchée sur carbonate anhydre de potassium.
- La solution filtrée puis évaporée nous donne une huile jaune qui est redissoute dans 40 ml AcoET.
- Cette solution est placée au frigo pendant une nuit.
- Les cristaux formés sont filtrés, lavés à l'hexane, séchés.
- On obtient 7,0 gr d'un produit blanc-crème (rendement = 90 %).

IR = 3260, 3090, 3050
1630, 1550 ;
1485, 1440, 760, 750, 740.

NMR $^1H$ S1.23 (br S, 2H, $NH_2$)
2.63 (m, 2H, $CH_2NH_2$)
2,99 (m, 2H, $NHCH_2$)
3,13 (S, 2H, $COCH_{2S)}$
6,36 (m, 1H, CONH)
7,1-7,5 (m, 15H,aryl)

2. Synthèse du N-[2-(N-(4-) pentene-3-one-2) aminoéthyl]-2-(triphenylméthylthio) acétamide

2.1.

- Sous azote sec ; 1,88 gr de 2 (5 mM) sont dissous dans 15 ml d'acétonitrile contenant 0,5 gr de tamis moléculaire. A cette solution, on ajoute 0,6 ml (6 mM) d'acétylacétone et on laisse réagir la nuit à température ambiante.

2.2.

- Après filtration et évaporation sous vide, le mélange réactionnel fournit une huile jaune qui est purifiée sur silice avec comme éluant un gradient de méthanol dans le dichlométhane. On obtient finalement 2,2 gr d'un solide jaune.

2.3. La recristallisation dans un mélange d'acétate d'éthyle et de diisopropyléther donne 1,52 gr de cristaux blanc. (rendement = 65 %).

2.4.

NMR § 1,63 (brS, 2H $NH_2$)
1,88 (S, 3H $-CH_3$) 7,1-7,5/m, 15H, aryl)

2,01 (S, 3H ($CH_3$)
3,05 (m, 2H $NHCH_2$)
3,13 (S, 2H $COCH_2S$)
3,18 (m, 2H $CH_2NH$)

Spectrométrie de masse

$M^+$ = M/z458 1 %
M/z243 100 %
M/z215 28 %
M/z165 37 %

**EXEMPLE 6: Synthèse de N[4-(pentène-3-one)]N'(2-pyrrolylméthyl) glycinamide (ci-après MRP26)**

Le composé MRP26 répond à la formule suivante :

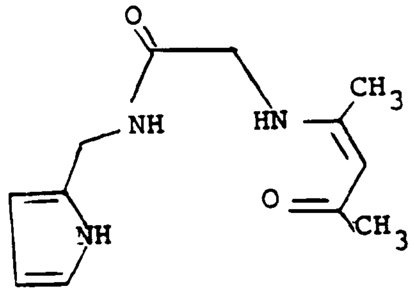

Le schéma réactionnel est le suivant :

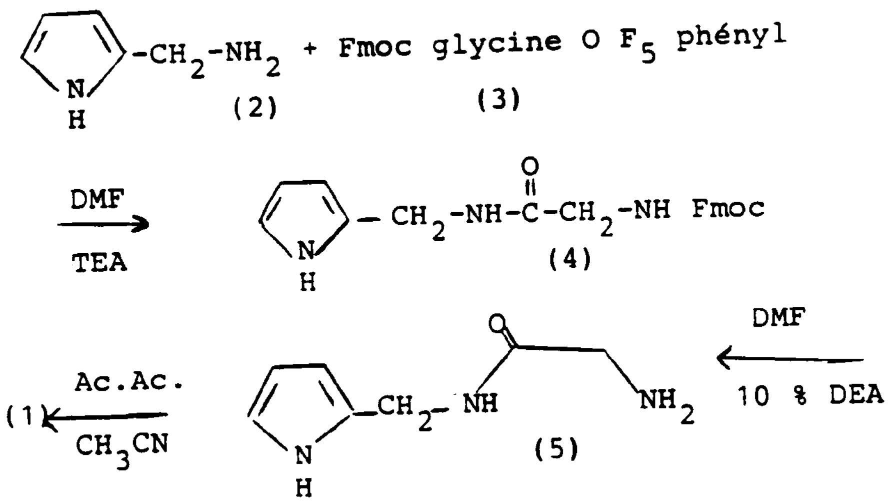


1. Synthèse N Fmoc N' (2 pyrrolyl méthyl) glycinamide (4)
   1.1.
      - 200 mg de 2 pyrrolyl aminométhyl (2,09 mM) sont dissous dans 10 ml DMF contenant 0,16 ml (2,2 mM) de triéthylamine.
      - On ajoute rapidement 1 g (2,09 mM) de Fmoc glyc $OF_5$ phényl.
      - On agite sous $N_2$ pendant 1 h.

   1.2. - On concentre sous vide puis on extrait l'huile restante dans un mélange eau - dichlorométhane.
   1.3. L'huile jaunâtre résultant de l'évaporation des phases organiques est recristallisée dans $CH_2Cl_2$ - Hexane. On obtient finalement 0,77 g (1,9 mM) de cristaux crème (rendement d'environ 90 %).
   1.4. La déprotection s'effectue dans 9 ml DMF + 1 ml DEA. Après 1 h, on évapore sous vide le maximum de solvant. Le produit brut obtenu est engagé rapidement dans l'étape de condensation.
2. Synthèse de N[4-(pentene-3-one-2)]N'(2 pyrrolylméthyl) glycinamide
   2.1.
      - Sous $N_2$, (4) brut est mis en solution dans 10 ml de $CH_3CH$.
      - On ajoute 0,4 ml (4 mM) d'acétyl acétone.
      - Après 15', un précipité blanc se forme. On maintient le ménage réactionnel sous agitation pendant la nuit.

   2.2. On évapore sous vide le maximum de solvant.
   On purifie le produit brut sur une colonne de silice avec comme éluant 0,5 % DEA dans l'acétate d'éthyle.
   On isole 0,4 g d'un solide légèrement jaune.
   2.3. On le recristallise dans 15 ml d'acétate d'éthyle.
   On obtient 0,26 g de cristaux blancs.

(rendement final ) 50 %)
TLC $CH_2Cl_2$ 95/MeOH 5
AcOEt/0,5 % DEA
NMR (ppm) 10,8 (s,bv) ; 9,4 (S, br) ; NH
6,75 (qu) ; 6,12 (qu), 6,01 (tr) pyrrole
5,1 (S)CH ; 4,35 (d) $CH_2$ ; 3,9 (d)$CH_2$
1,85 (s) ; 2,05 (s)$CH_3$, $CH_3$ ; 1,6 (S) NH
MASSE (EI, 70 ev)

$M^+$ M/Z 235 ; fragments → 135, 112, 100, 94, 80

| | | Theor. | Trouve |
|---|---|---|---|
| CHN | C- | 61,28 | 59,86 |
| | H- | 7,29 | 7,18 |
| | N- | 17,87 | 17,45 |

**EXEMPLE 7: SYNTHESE DU COMPOSE MRP210 DE FORMULE SUIVANTE**

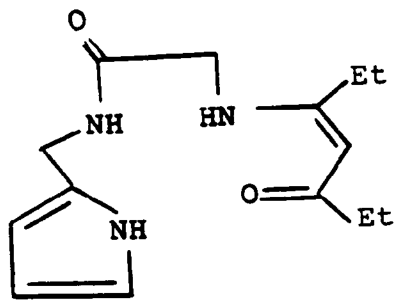

qui correspond à 1 a formule VI dans laquelle
$R_1 = R_3$ = éthyl
$R_2 = R_4 = R_5$ = H
X = pyrolyl

Le composé a été préparé suivant un protocole similaire à celui décrit à l'exemple 9.

RMN Déplacement en ppm.

| | | |
|---|---|---|
| 10,84 | (s.br) | NH |
| 9,00 | (s.br) | |
| 6,71 | (qu) | pyrolle |
| 6,08 | (qu) | |
| 6,00 | (s) | |
| 5,14 | (s) | CH |
| 4,36 | (d) | $CH_2$ |
| 3,93 | (d) | |
| 2,32 | (qu) | |
| 2,14 | (qu) | |
| 1,5 | (qu) | $CH_3$ |

Masse (EI, 70 ev)

$M^+$ M/Z = 2,63
Fragments → 189, 136, 128, 117, 93, 72

**EXEMPLE 8 : COMPLEXE DE TECHNETIUM**

Les ligands décrits ci-dessus réagissent dans une solution éthanolique-saline avec du pertechnétate (obtenu d'un générateur commercial) et en présence d'un agent réducteur (avec ou sans base ou agent stabilisant comme l'acide paraaminobenzoïque) pour donner des complexes de technétium-ligand. Les agents réducteurs peuvent être choisis parmi les suivants : étain (II), dithionite de sodium, acide formamidine-sulphinique, hydrazine ou tout autre réducteur usuel avec un potentiel d'oxydoréduction approprié.

1) Synthèse du complexe de N[-2(-1H-pyrrolylméthyl)]N'[-4-(pentène-3-one-2)] éthylène 1,2 diamine et $^{99m}Tc$

Dans un flacon de 10 ml, introduire successivement 4 mg de ligand, 1 ml d'éthanol, 1 ml d'une solution aqueuse saline (NaCl 0,9 %), un maximum de 0,5 ml d'une solution aqueuse saline de $^{99m}TcO^-_4$ et 20 µg de $SnCl_2$ fraîchement dissout dans de l'eau dégazée. Laisser réagir 30 min à température ambiante. L'analyse des produits de réaction par CCM sur gel de silice (ITLC-SG) montre la présence d'un produit lipophile qui migre avec MEK (méthyléthylcétone) et ne migre pas avec une solution aqueuse saline. Le composé lipophile peut être chromatographié par HPLC et est retenu sur un support chromatographique en phase inverse.

2) Synthèse du complexe 1) en présence d'une base non coordinante

Dans une solution de 10 ml, introduire successivement 4 mg de ligand, 5 mg de 1,2'-bipyridyl, 1 ml d'éthanol, 1 ml d'une solution aqueuse saline, un maximum de 0,5 ml d'une solution de $^{99m}TcO^-_4$ et 20 µg de $SnCl_2$ fraîchement dissout dans de l'eau dégazée. La réaction est effectuée comme à l'exemple 3.1).

3) Synthèse du complexe 1) en présence d'ions hydroxyle

Dans un flacon de 10 ml, introduire successivement 4 mg de ligand, 1 ml d'éthanol, 1 ml d'une solution aqueuse saline. Ajuster le pH à 9,5 au moyen de NaOH. Ajouter un maximum de 0,5 ml de $^{99m}TcO^-_4$ et 20 µg de $SnCl_2$. La réaction est effectuée comme à l'exemple 3.1).

4) Synthèse de complexe 1) en présence d'acide paraaminibenzoïque

Dans un flacon de 10 ml, introduire successivement 4 mg de ligand, 80 µg d'acide paraaminobenzoïque, 1 ml d'éthanol, 1 ml d'une solution aqueuse saline, un maximum de 0,5 ml d'une solution saline de $^{99m}TcO^-_4$ et 20 µg de $SnCl_2$. La réaction est effectuée comme à l'exemple 3.1).

5) Synthèse de complexe 1) avec du tartrate d'étain

Dans un flacon de 10 ml, introduire successivement 4 mg de ligand, 1 ml d'éthanol, 1 ml d'une solution aqueuse saline, un maximum de 0,5 ml d'une solution aqueuse saline de $^{99m}TcO^-_4$ et 50 µg de tartrate d'étain (II) en solution. La réaction est effectuée comme pour l'exemple 3.1).

6) Synthèse du complexe de N[-2(-1H-pyrrolylméthyl)N'[-4(-5-trifluoropentène-3-one-2)] éthylène 1,2 diamine et de $^{99m}Tc$

En utilisant 4 mg de ligand, effectuer la réaction comme dans l'exemple 3.1). Le temps de rétention du composé lipophile obtenu par HPLC est différent de celui du complexe 1).

7) Synthèse du complexe 1) à différentes températures

Dans un flacton de 10 ml, introduire successivement 4 mg de ligand, 1 ml d'éthanol, 1 ml d'une solution aqueuse saline, un maximum de 0,5 ml d'une solution aqueuse saline de $^{99m}TcO^-_4$ et 20 µg de $SnCl_2$. Mettre dans un bain d'eau à 20°C, 40°C et 100°C pendant 30 min. Effectuer les analyses comme à l'exemple 3.1).

8) Synthèse du complexe 1) en variant les quantités de réactifs

a) Effectuer la réaction décrite dans l'exemple 3.1) en utilisant 1,4 mg de ligand à la place de 4 mg de ligand.

b) Effectuer la réaction décrite dans l'exemple 3.1) en utilisant 5, 10, 20 et 50 µg de $SnCl_2$.

**EXEMPLE 9 : BIODISTRIBUTION DU COMPLEXE MRP20**

Une étude de biodistribution a été effectuée sur des rats femelles Wistar en utilisant le complexe $^{99m}Tc$ - N[-2(-1H-pyrrolylméthyl)] N'[-4(pentène-3-one-2)] éthylène 1,2 diamine (ci-après MRP20). Les animaux ont subi des injections dans la veine fémorale et ont été tués à différentes périodes après l'injection : 5 min, 15 min, 30 min, 60 min et 180 min. Trois animaux ont été sacrifiés à ces différentes périodes. Les organes d'intérêt ont été retirés, nettoyés, débarassés de leur sang et on a procédé à une évaluation scintigraphique à l'aide d'un compteur gamma à partir d'un échantillon standard préparé à partir de la solution injectée. Les pourcentages de doses injectées par organe et par gramme de tissus ont été calculés et sont présentés aux tableaux

1 et 2, un graphique de clairance est présenté dans la figure 1.

Bien que la fixation initiale dans le cerveau paraisse être lente, le complexe est retenu pendant trois heures et atteint un maximum de 2,35 % de la dose injectée par organe. Ce pourcentage est très safisfaisant compte tenu de ce qui est couramment admis pour un traceur cérébral, à savoir 2 à 3 % de fixation dans le cerveau du rat, ce qui correspond en général pour des primates à un taux de fixation supérieur à 4-5 %.

TABLEAU 1 : Pourcentage de la dose injectée par organe

| ORGANE | MR20/RAT/DOSE/ORGANE | | | | |
|---|---|---|---|---|---|
| | 5 min | 15 min | 30 min | 60 min | 180 min |
| sang | 14,70 | 11,58 | 13,22 | 12,44 | 9,27 |
| cerveau | 1,05 | 0,77 | 2,35 | 2,08 | 1,68 |
| coeur | 1,11 | 0,86 | 1,43 | 1,31 | 0,88 |
| foie | 5,9 | 4,08 | 13,2 | 8,98 | 4,27 |
| reins | 2,41 | 3,66 | 5,84 | 6,45 | 7,95 |
| poumon | 1,97 | 1,23 | 2,21 | 2,05 | 1,31 |
| estomac | 0,88 | 1,14 | 2,85 | 2,51 | 1,73 |
| intestin | 2,69 | 5,67 | 9,32 | 9,89 | 15,27 |

TABLEAU 2 : Pourcentage de la dose injectée par gramme de tissus

| ORGANE | MRP20/DOSE/GRAMME | | | |
|---|---|---|---|---|
| | 5 min | 30 min | 60 min | 180 min |
| sang | 1,49 | 1,14 | 1,10 | 0,77 |
| cerveau | 0,97 | 1,74 | 1,31 | 1,34 |
| coeur | 2,18 | 2,98 | 2,07 | 1,64 |
| foie | 0,65 | 2,48 | 1,34 | 0,78 |
| reins | 1,69 | 4,74 | 4,65 | 5,92 |
| poumon | 2,4 | 2,5 | 1,79 | 1,62 |
| estomac | 0,36 | 0,92 | 0,79 | 0,79 |
| intestin | 0,12 | 0,92 | 0,68 | 1,32 |

**EXEMPLE 10: BIODISTRIBUTION DES COMPLEXES MRP21, MRP22, MRP30, MRP27 ET MRP26**

Une étude de biodistribution a été effectuée sur des rats femmelles wistar en utilisant des complexes du $^{99m}Tc$ de différents composés suivant le protocole suivant.

Les animaux ont subi des injections dans la veine fémorale et ont été tués à différentes périodes après l'injection : 5 min, 15 min, 30 min, 60 min et 180 min. Trois animaux ont été sacrifiés à ces différentes périodes.

Les organes d'intérêt ont été retirés, nettoyés, débarassés de leur sang et on a procédé à une évaluation scintigraphique à l'aide d'un compteur gamma à partir d'un échantillon standard préparé à partir de la solution injectée. Les pourcentages de doses injectées par organe ont été calculés et sont présentés aux tableaux 1 à 3, un graphique de clairance est présenté dans la figure 2.

1) Composé MRP22 de formule

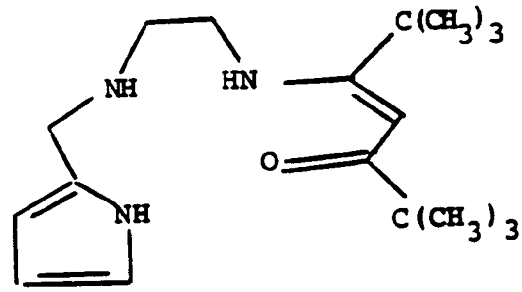

TABLEAU 1 :

| ORGANE | MRP22/RAT/DOSE/ORGANE | | | | |
|---|---|---|---|---|---|
| | 5 min | 15 min | 30 min | 60 min | 180 min |
| reins | 12,74 | 11,21 | 9,35 | 9,29 | 8,08 |
| foie | 22,12 | 23,65 | 22,06 | 15,53 | 8,24 |
| estomac | 0,69 | 0,91 | 0,91 | 1,63 | 1,76 |
| poumons | 0,84 | 0,51 | 0,32 | 0,32 | 0,25 |
| muscles | 0,15 | 0,05 | 0,05 | 0,02 | 0,01 |
| coeur | 0,38 | 0,13 | 0,10 | 0,07 | 0,04 |
| rate | 0,20 | 0,10 | 0,08 | 0,10 | 0,08 |
| intestin | 4,27 | 11,83 | 14,86 | 16,64 | 28,98 |
| cerveau | 0,17 | 0,12 | 0,10 | 0,07 | 0,04 |
| sang | 13,24 | 4,20 | 2,64 | 1,79 | 1,04 |

2) Composé MRP30 de formule

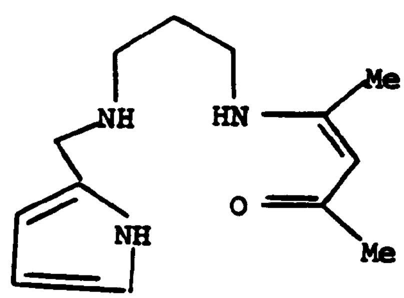

TABLEAU 2

| ORGANE | MRP30/RAT/DOSE/ORGANE | | |
|---|---|---|---|
| | 5 min | 30 min | 3 heures |
| cerveau | 0,12 | 0,03 | 0,01 |
| coeur | 0,41 | 0,25 | 0,12 |
| sang | 24,45 | 15,16 | 6,62 |
| foie | 7,63 | 6,25 | 3,53 |
| reins | 2,04 | 2,13 | 2,77 |
| estomac | 6,11 | 16,62 | 23,04 |
| intestin | 5,95 | 8,21 | 17,3 |
| poumons | 4,59 | 3,32 | 1,15 |

3) Composé MRP21 de formule

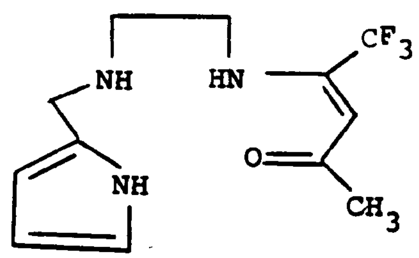

TABLEAU 3

| ORGANE | MRP21/RAT/DOSE/ORGANE | | | |
|---|---|---|---|---|
| | 5 min | 30 min | 1 heure | 3 heures |
| sang | 10,12 | 7,15 | 7,56 | 5,26 |
| cerveau | 1,05 | 0,93 | 1,06 | 0,77 |
| coeur | 0,74 | 0,55 | 0,61 | 0,40 |
| foie | 13,94 | 12,73 | 13,68 | 5,68 |
| reins | 2,53 | 3,58 | 4,63 | 4,43 |
| poumons | 1,22 | 0,98 | 1,15 | 0,70 |
| estomac | 1,29 | 1,39 | 2,17 | 1,40 |
| intestins | 4,6 | 7,99 | 17,69 | 17,04 |
| glandes surrenales | 0,21 | 0,14 | 0,12 | 0,08 |
| thymus | 0,26 | 0,20 | 0,22 | 0,1 |

4) Composé MRP27 de formule

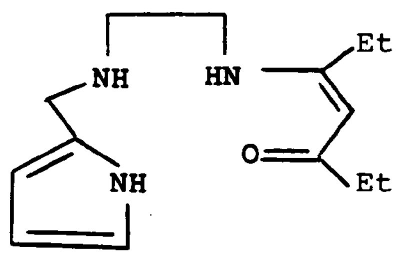

La figure 3 qui représente le pourcentage de dose injectée par organe au cours du temps dans le rat montre qu'une captation dans le cerveau similaire au complexe MRP20.

L'épuration dans le sang est aussi bonne avec le MRP27 que le MRP20.

5) Complexe MRP26

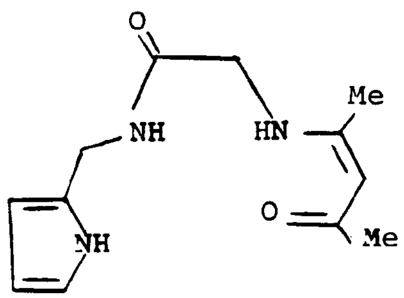

La figure 4 qui représente un graphique de biodistribution dans le rat d'un complexe $^{99m}Tc$ MRP26 montre notamment une élimination du foie très rapide.

## Revendications

**Revendications pour les Etats contractants suivants : AT, BE, CH, DE, FR, DK, GB, GR, IT, LI, LU, NL, SE**

**1.** Composés répondant à la formule générale suivante :

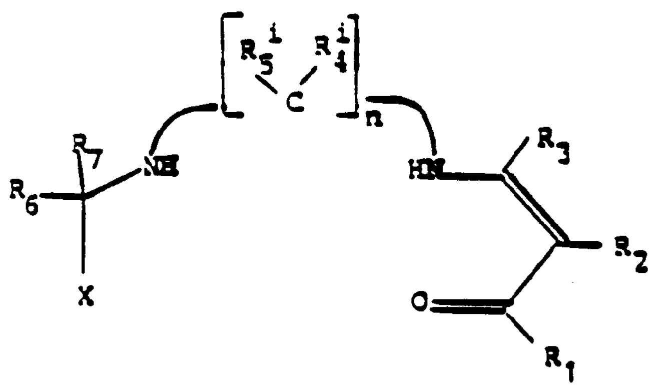

I

dans laquelle

$R_1$ et $R_3$ représentent indépendamment l'un de l'autre H, un groupe alkyle, alkényle, cycloalkyle ayant 5, 6 ou 7 atomes de carbone, aryle, arylalkyle, un alkylaryle, un hétérocycle de 5 ou 6 chaînons contenant au moins un atome d'azote, groupes éventuellement substitués, notamment par un ou des groupe(s) hydroxy, alcoxy ou halogène ;

n représente un nombre entier de 1 à 5 ;

i prend respectivment les valeurs de 1 à n pour les n maillons successifs

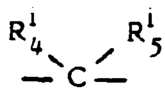

$R_2$, $R_4^i$ et $R_5^i$ représentent indépendamment l'un de l'autre H, un groupe alkyle, alkényle, aryle, alcoxy, hydroxyalkyle, alcoxyalkyle, amide, acyle ou carboxyalkyle, ou un sel, ou un ester alkylique de ce dernier ; ou $R_4^i$et $R_5^i$forment ensemble un groupe oxo

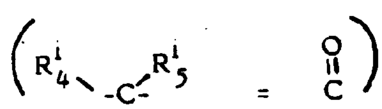

$R_6$, $R_7$ représentent H,

X représente un hétérocycle de 5 ou 6 chaînons contenant au moins un atome d'azote,

dans $R_1$, $R_2$, $R_3$, $R_4^i$ et $R_5^i$:

- les termes acyle, alkyle, hydroxy-, carboxy- ou alcoxy-alkyle et alcoxy se réfèrent à des chaînes droites ou ramifiées de 1 à 10 atome(s) de carbone ;
- les groupes alkényle sont constitués de chaînes droites ou ramifiées de 2 à 10 atomes de carbone ;
- le terme aryle se réfère au groupe phényl.

**2.** Composés selon la revendication 1, caractérisés en ce que X est un des composés représentés par la formule II ou un de leurs dérivés déhydro :

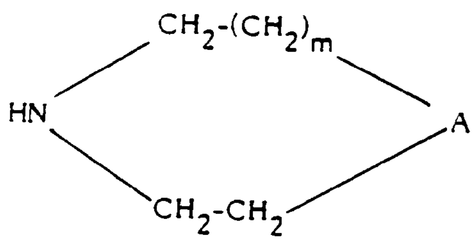

II

dans laquelle

m est 0 ou 1 et

A est O, N-$R_{11}$ ou CH-$R_{11}$ où $R_{11}$ représente H, alkyle, aryle, éventuellement substitués.

3. Composés selon la revendication 2, caractérisés en ce que X est un groupe pyrrolyle, imidazolyle, oxazolyle, pyrazolyle, pyridinyle, pyrimidinyle.

4. Composés selon l'une des revendications 1 à 3, représentés par la formule I caractérisés en ce que
   . X =

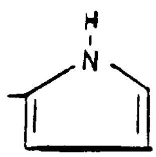 , 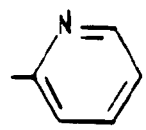 ,

avec
. n = 2 ou 3
. $R_4^i = R_5^i$ = H avec i = 1 à n ou 1 à n-1
. $R_1$ et $R_3$ représentent indépendamment l'un de l'autre H, $CH_3$, $CF_3$, $C_2H_5$, $C(CH_3)_3$ et $R_2$ = H

5. Composés selon l'une des revendications 1 à 4, répondant à la formule III :

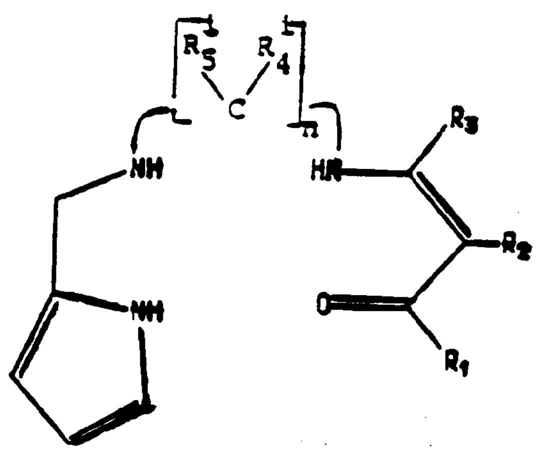

III

dans laquelle $R_1$, $R_2$, $R_3$, $R_4^i$ et $R_5^i$ ont les significations données précédemment.

6. Composés selon l'une des revendications 1 à 4, répondant à la formule IV :

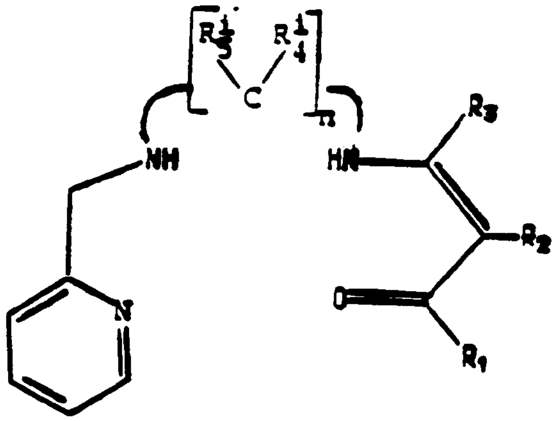

IV

dans laquelle $R_1$, $R_2$, $R_3$, $R_4^i$, $R_5^i$ ont les significations données précédemment pour la formule générale I.

7. Composé selon l'une des revendications 1 à 4 répondant à la formule VI

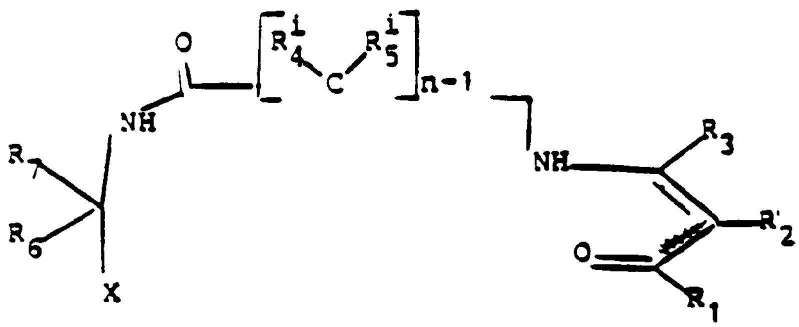

VI

dans laquelle $R_1$ à $R_7$ ont les significations données précédemment pour la formule générale I.

8. Composé selon la revendication 7 répondant à la formule VI, caractérisé en ce que $R_6 = R_7 = H$, X = hétérocycle, $R_4^i = R_5^i = H$, i = 1 à n-1.

9. Procédé de préparation de composé selon l'une des revendications 1 à 8, caractérisé en ce qu'on fait réagir un composé de formule VII

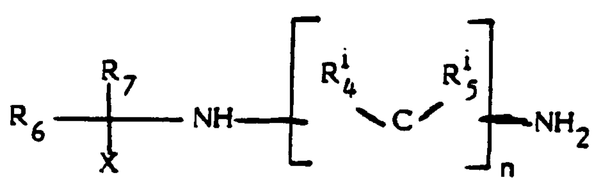

VII

avec une dione de formule VIII

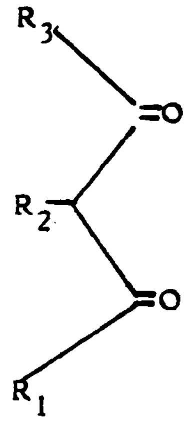

10. Procédé de préparation selon la revendication 9, caractérisé en ce qu'on prépare le composé de formule VII en faisant réagir un hétérocycle X substitué par un groupe formyle de formule

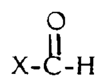

avec une fonction amine d'un alkylène diamine de formule $NH_2$-$(CR'_4 R'_5)_n$-$NH_2$ dans de l'acétonitrile en présence de $NaBH_4$.

11. Procédé selon la revendication 10, caractérisé en ce que lorsque

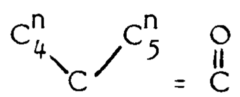

on fait réagir l'amine X $(CR_6R_7)NH_2$ avec l'amino-acide $HOOC(CR'_4R'_5)_{n-1}$-$NH_2$ en activant la fonction acide par un groupe activateur conventionnel dans la chimie peptidique et en protégeant la fonction $NH_2$ par un groupe protecteur conventionnel.

12. Complexe de coordination d'un composé selon l'une des revendications 1 à 8 avec un métal ou un ion métallique, radioactif ou paramagnétique.

13. Complexe de coordination selon la revendication 12, caractérisé en ce que le métal $^{111}In$, $^{113m}In$, $^{67}Ga$, $^{68}Ga$, $^{157}Gd$, $^{201}Ti$, $^{117m}Sn$, $^{64}Cu$, $^{186}Re$, $^{188}Re$, $^{90}Y$, $^{212}Bi$, $^{99}Tc$, $^{99m}Tc$, $^{97}Ru$, $^{51}Cr$, $^{57}Co$, $^{191}Os$.

14. Complexe de coordination selon la revendication 13, caractérisé en ce que le métal est le $^{99m}Tc$.

15. Kit constitué par un ligand consistant en un composé selon l'une des revendications 1 à 8, et de réactifs permettant la formation de complexes métalliques selon les revendications 12, 13 et 14 lorsque ledit kit est reconstitué en présence de composés desdits métaux.

16. Kit selon la revendication précédente, caractérisé en ce que le composé de métal est le pertechnétate de $^{99m}Tc$, les réactifs conduisant à la formation de complexe de coordination comprennent un agent réducteur, tel qu'un sel d'étain.

17. Kit selon l'une des revendications précédentes, caractérisé en ce que le composé selon les revendications 1 à 8 est en solution hydroalcoolique ou sous forme lyophilisée.

18. Kit selon la revendication 15, caractérisé en ce que les réactifs comprennent également un agent stabilisant comme l'acide paraaminobenzoïque ou une base.

19. Kit selon l'une des revendications précédentes utile pour l'imagerie médicale du cerveau, du coeur, des infections ou le marquage des lymphocytes pour l'imagerie des sites inflammatoires.

**20.** Kit destiné à l'imagerie du cerveau ou au marquage des globules blancs selon l'une des revendications précédentes, caractérisé par le fait que le composé métallique utilisé pour reconstituer le kit est le pertechnétate $^{99m}TcO^{-}_{4}$.

**Revendications pour l'Etat contractant suivant : ES**

**1.** Procédé de préparation de composés répondant à la formule générale suivante:

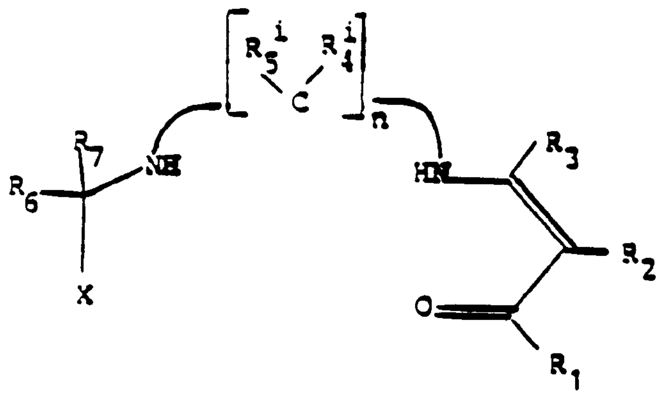

I

dans laquelle

R$_1$ et R$_3$ représentent indépendamment l'un de l'autre H, un groupe alkyle, alkényle, cycloalkyle ayant 5, 6 ou 7 atomes de carbone, aryle, arylalkyle, un alkylaryle, un hétérocycle de 5 ou 6 chaînons contenant au moins un atome d'azote, groupes éventuellement substitués, notamment par un ou des groupe(s) hydroxy, alcoxy ou halogène ;

n représente un nombre entier de 1 à 5 ;

i prend respectivement les valeurs de 1 à n pour les n maillons successifs

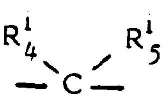

$R_2$, $R^i_4$ et $R^i_5$ représentent indépendamment l'un de l'autre H, un groupe alkyle, alkényle, aryle, alcoxy, hydroxyalkyle, alcoxyalkyle, amide, acyle ou carboxyalkyle, ou un sel, ou un ester alkylique de ce dernier ; ou $R^i_4$ et $R^i_5$ forment ensemble un groupe oxo

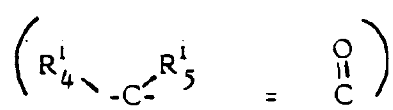

$R_6$, $R_7$ représentent H,

X représente un hétérocycle de 5 ou 6 chaînons contenant au moins un atome d'azote,

dans $R_1$, $R_2$, $R_3$, $R^i_4$ et $R^i_5$:

- les termes acyle, alkyle, hydroxy-, carboxy- ou alcoxy-alkyle et alcoxy se réfèrent à des chaînes droites ou ramifiées de 1 à 10 atome(s) de carbone ;
- les groupes alkényle sont constitués de chaînes droites ou ramifiées de 2 à 10 atomes de carbone ;
- le terme aryle se réfère au groupe phényl,

caractérisé en ce qu'on fait réagir un composé de formule VII

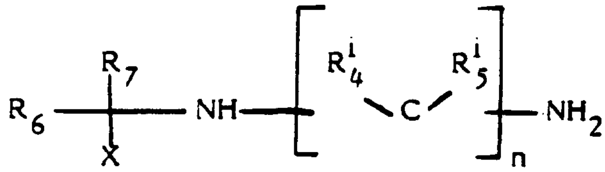 VII

avec une dione de formule VIII

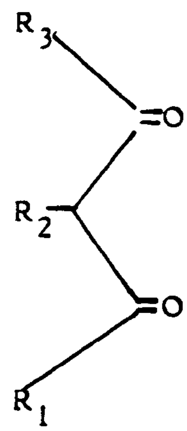

2. Procédé de préparation de composés selon la revendication 1, caractérisé en ce que X est un des composés représentés par la formule II ou un de leurs dérivés déhydro :

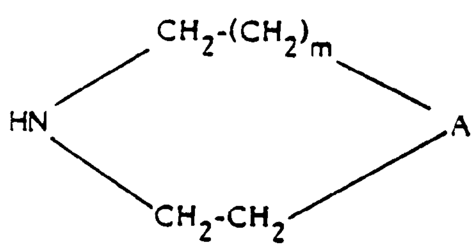 II

dans laquelle

m est 0 ou 1 et

A est O, N-$R_{11}$ ou CH-$R_{11}$ où $R_{11}$ représente H, alkyle, aryle, éventuellement substitués.

3. Procédé de préparation de composés selon la revendication 2, caractérisé en ce que X est un groupe pyrrolyle, imidazolyle, oxazolyle, pyrazolyle, pyridinyle, pyrimidinyle.

4. Procédé de préparation de composés selon l'une des revendications 1 à 3, représentés par la formule I, caractérisé en ce que

. X =

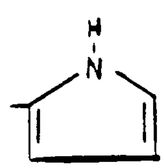 , 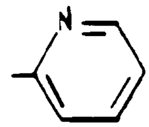 ,

avec

. n = 2 ou 3

. $R_4^i = R_5^i$ = H avec i = 1 à n ou 1 à n-1

. $R_1$ et $R_3$ représentent indépendamment l'un de l'autre H, $CH_3$, $CF_3$, $C_2H_5$, $C(CH_3)_3$ et $R_2$ = H

5. Procédé de préparation de composés selon l'une des revendications 1 à 4, répondant à la formule III :

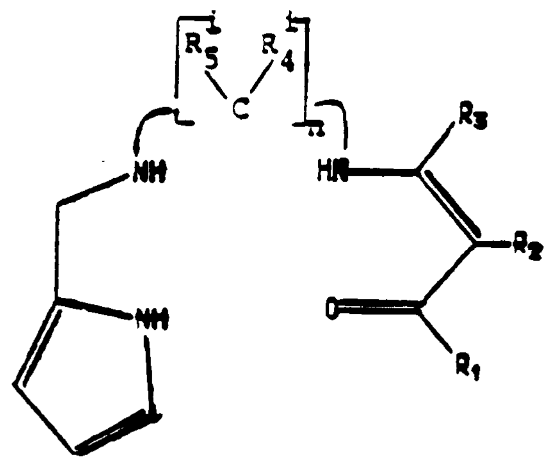

III

dans laquelle $R_1$, $R_2$, $R_3$, $R^i_4$ et $R^i_5$ ont les significations données précédemment.

6. Procédé de préparation de composés selon l'une des revendications 1 à 4, répondant à la formule IV :

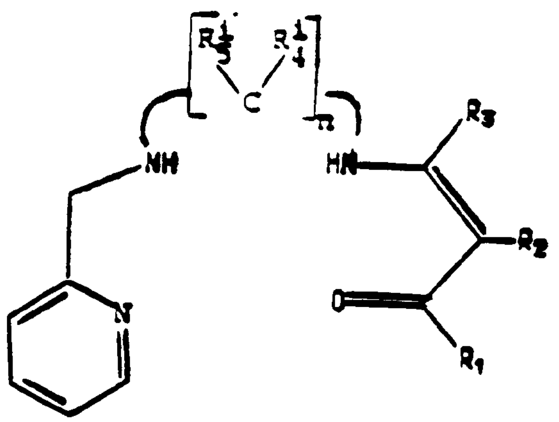

IV

dans laquelle $R_1$, $R_2$, $R_3$, $R^i_4$, $R^i_5$ ont les significations données précédemment pour la formule générale I.

7. Procédé de préparation de composé selon l'une des revendications 1 à 4, répondant à la formule VI

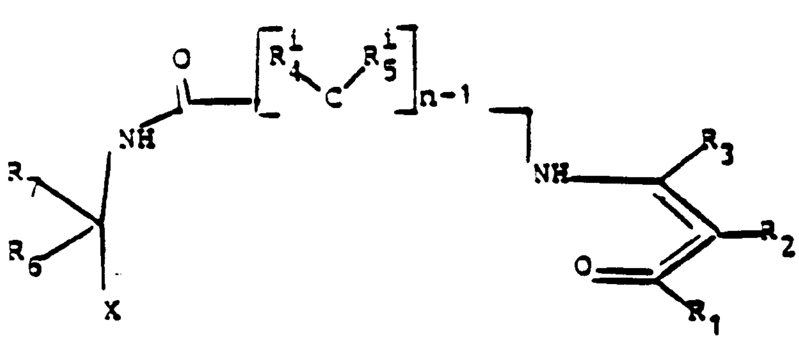

VI

dans laquelle $R_1$ à $R_7$ ont les significations données précédemment pour la formule générale I.

8. Procédé de préparation de composé selon la revendication 7, répondant à la formule VI, caractérisé en ce que $R_6 = R_7 = H$, X = hétérocycle, $R^i_4 = R^i_5 = H$, i = 1 à n-1.

**9.** Procédé de préparation selon la revendication 8, caractérisé en ce qu'on prépare le composé de formule VII en faisant réagir un hétérocycle X substitué par un groupe formyle de formule

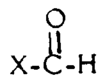

avec une fonction amine d'un alkylène diamine de formule $NH_2$-$(CR_4^i R_5^i)_n$-$NH_2$ dans de l'acétonitrile en présence de $NaBH_4$.

**10.** Procédé selon la revendication 9, caractérisé en ce que lorsque

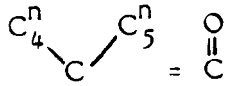

on fait réagir l'amine X $(CR_6R_7)NH_2$ avec l'amino-acide $HOOC(CR_4^iR_5^i)_{n-1}$-$NH_2$ en activant la fonction acide par un groupe activateur conventionnel dans la chimie peptidique et en protégeant la fonction $NH_2$ par un groupe protecteur conventionnel.

**11.** Procédé de préparation d'un composé selon l'une des revendications 1 à 8 avec un métal ou un ion métallique, radioactif ou paramagnétique

**12.** Procédé de préparation d'un complexe de coordination selon la revendication 11, caractérisé en ce que le métal $^{111}In$, $^{113m}In$, $^{67}Ga$, $^{68}Ga$, $^{157}Gd$, $^{201}Ti$, $^{117m}Sn$, $^{64}Cu$, $^{186}Re$, $^{188}Re$, $^{90}Y$, $^{212}Bi$, $^{99m}Tc$, $^{97}Ru$, $^{51}Cr$, $^{57}Co$, $^{191}Os$.

**13.** Procédé de préparation d'un complexe de coordination selon la revendication 12, caractérisé en ce que le métal est le $^{99m}Tc$.

## Claims

### Claims for the following Contracting States : AT, BE, CH, DE, DK, FR, GB, GR, IT, LI, LU, NL, SE

**1.** A compound which corresponds to the following general formula:

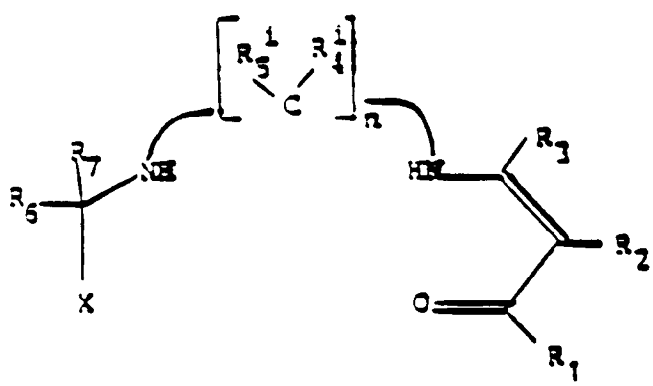

I

in which

$R_1$ and $R_3$ independently of one another represent H, an alkyl or alkenyl group, a cycloalkyl group having 5,6 or 7 carbon atoms, or an aryl, arylalkyl or an alkylaryl group, a 5- or 6-membered heterocycle which contains at least one nitrogen atom, or groups which are unsubstituted or substituted, in particular by one or more hydroxyl or alkoxy groups, or halogen;

| | |
|---|---|
| n | represents an integer from 1 to 5; |
| i | in each case takes values from 1 to n for the n successive |

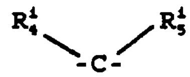

links;

| | |
|---|---|
| $R_2$, $R_4^i$ and $R_5^i$ | independently of one another represent H, an alkyl, alkenyl, aryl, alkoxy, hydroxyalkyl, alkoxyalkyl, amido, acyl or carboxyalkyl group, or a salt, or an alkyl ester of the latter; or $R_4^i$ and $R_5^i$ together form an oxo group |

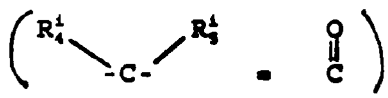 ;

| | |
|---|---|
| $R_6$ and $R_7$ | represent H; |
| X | represents a 5- or 6-membered heterocycle which contains at least one nitrogen atom; |

in $R_1$, $R_2$, $R_3$, $R_4^i$, and $R_5^i$:

- the terms acyl, alkyl, hydroxyalkyl, carboxyalkyl, alkoxyalkyl and alkoxy denote straight or branched chains having 1 to 10 carbon atoms;
- the alkenyl groups consist of straight or branched chains having 2 to 10 carbon atoms;
- the term aryl denotes the phenyl group.

**2.** The compound as claimed in claim 1, wherein X is one of the compounds represented by the formula II or one of the dehydro derivatives thereof:

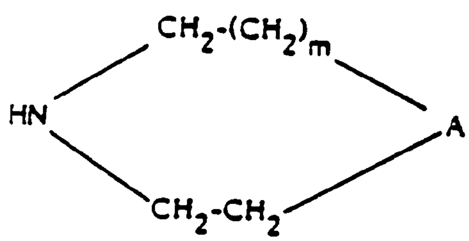 II

in which

| | |
|---|---|
| m | is 0 or 1 and |
| A | is O, $N-R_{11}$ or $CH-R_{11}$, where $R_{11}$ represents H or substituted or unsubstituted alkyl or aryl. |

**3.** The compound as claimed in claim 2, wherein X is a pyrrolyl, imidazolyl, oxazolyl, pyrazolyl, pyridinyl or pyrimidinyl group.

**4.** The compound as claimed in one of claims 1 to 3, represented by the formula I, wherein

. X =

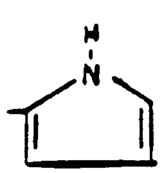 , 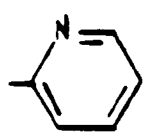 ,

where

. n is 2 or 3,

. $R_4^i = R_5^i = H$ where i = 1 to n or 1 to n-1,

. $R_1$ and $R_3$ independently of one another represent H, $CH_3$, $CF_3$, $C_2H_5$ and $C(CH_3)_3$, and $R_2$ = H.

5. The compound as claimed in one of claims 1 to 4, which corresponds to the formula III:

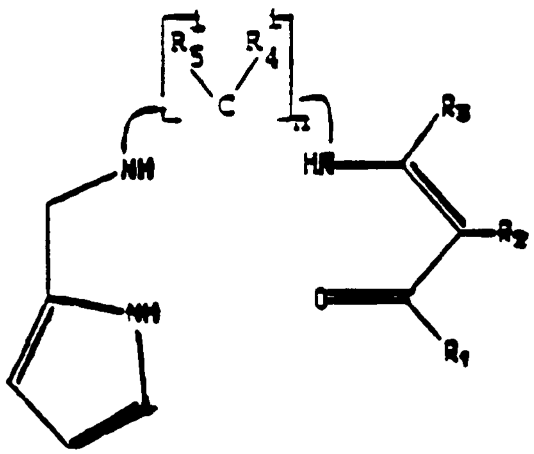

III

in which $R_1$, $R_2$, $R_3$, $R_4^i$ and $R_5^i$ have the meanings given above.

6. The compound as claimed in one of claims 1 to 4, which corresponds to the formula IV:

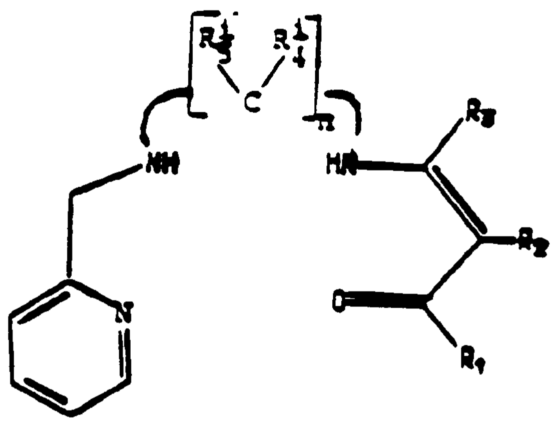

IV

in which $R_1$, $R_2$, $R_3$, $R_4^i$ and $R_5^i$ have the meanings given above in the case of the general formula I.

7. The compound as claimed in one of claims 1 to 4, which corresponds to the formula VI

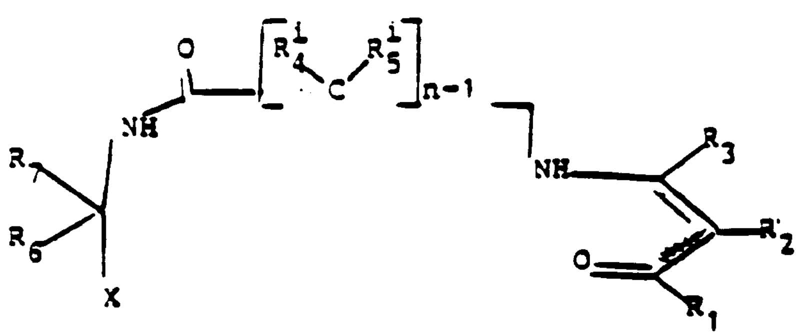

VI

in which $R_1$ to $R_7$ have the meanings given above in the case of general formula I.

8. The compound as claimed in claim 7, which corresponds to the formula VI, wherein $R_6$ = $R_7$ = H, X = het-

erocycle, $R_4^i = R_5^i = H$, and i = 1 to n-1.

**9.** A process for the preparation of a compound as claimed in one of claims 1 to 8, which comprises reacting a compound of the formula VII

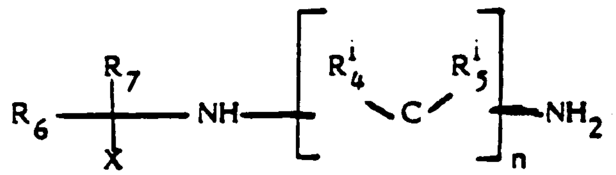

VII

with a dione of the formula VIII

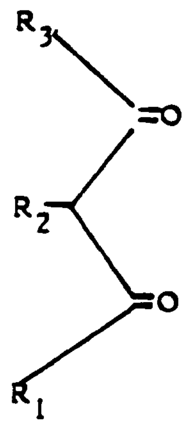

**10.** The preparation process as claimed in claim 9, wherein the compound of the formula VII is prepared by reacting a heterocycle X which is substituted by a formyl group of the formula

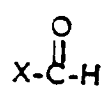

with an amine functional group of an alkylenediamine of the formula $NH_2$- $(CR_4^iR_5^i)_n$-$NH_2$ in acetonitrile in the presence of $NaBH_4$.

**11.** The process as claimed in claim 10, wherein, when

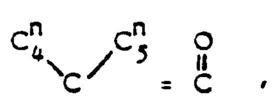

the amine $X(CR_6R_7)NH_2$ is reacted with the amino acid $HOOC(CR_4^iR_5^i)_{n-1}$-$NH_2$ by activating the acid functional group with an activating group customary in peptide chemistry and by protecting the $NH_2$ functional group by a customary protective group.

**12.** A coordination complex of a compound as claimed in one of claims 1 to 8, with a metal or a radioactive or paramagnetic metal ion.

**13.** The coordination complex as claimed in claim 12, wherein the metal is $^{111}In$, $^{113m}In$, $^{67}Ga$, $^{68}Ga$, $^{157}Gd$, $^{201}Ti$, $^{117m}Sn$, $^{64}Cu$, $^{186}Re$, $^{188}Re$, $^{90}Y$, $^{212}Bi$, $^{99}Tc$, $^{99m}Tc$, $^{97}Ru$, $^{51}Cr$, $^{57}Co$ and $^{191}Os$.

**14.** The coordination complex as claimed in claim 13, wherein the metal is $^{99m}Tc$.

15. A kit composed of a ligand which consists of a compound as claimed in one of claims 1 to 8, and of reagents which allow the formation of metal complexes as claimed in claims 12, 13 and 14 when said kit is constituted in the presence of the compounds of said metals.

16. The kit as claimed in the preceding claim, wherein the metal compound is $^{99m}Tc$ pertechnetate and the reagents which lead to the formation of a coordination complex comprise a reducing agent, such as a tin salt.

17. The kit as claimed in one of the preceding claims, wherein the compound as claimed in claims 1 to 8 is in an aqueous-alcoholic solution or in lyophilized form.

18. The kit as claimed in claim 15, wherein the reagents likewise comprise a stabilizer, such as paraaminobenzoic acid or a base.

19. The kit as claimed in one of the preceding claims which can be used for medical imaging of the brain, the heart and infections, or for labeling lymphocytes for imaging inflammatory sites.

20. The kit for the purpose of brain imaging or labeling white corpuscles as claimed in one of the preceding claims, wherein the metal compound used for constituting the kit is $^{99m}TcO_4^-$ pertechnetate.

**Claims for the following Contracting State : ES**

1. A process for the preparation of a compound which corresponds to the following general formula:

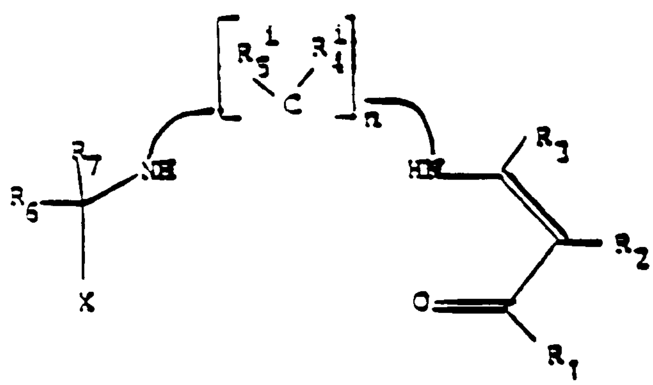

I

in which

R₁ and R₃ — independently of one another represent H, an alkyl or alkenyl group, a cycloalkyl group having 5,6 or 7 carbon atoms, or an aryl, arylalkyl or an alkylaryl group, a 5- or 6-membered heterocycle which contains at least one nitrogen atom, or groups which are unsubstituted or substituted, in particular by one or more hydroxyl or alkoxy groups, or halogen;

n — represents an integer from 1 to 5;

i — in each case takes values from 1 to n for the n successive

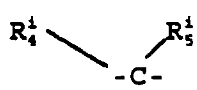

links;

$R_2$, $R_4^i$ and $R_5^i$ — independently of one another represent H, an alkyl, alkenyl, aryl, alkoxy, hydroxyalkyl, alkoxyalkyl, amido, acyl or carboxyalkyl group, or a salt, or an alkyl ester of the latter; or $R_4^i$ and $R_5^i$ together form an oxo group

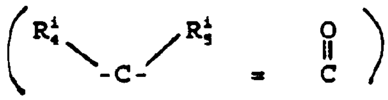 ;

| | |
|---|---|
| $R_6$ and $R_7$ | represent H; |
| X | represents a 5- or 6-membered heterocycle which contains at least one nitrogen atom; |

in $R_1$, $R_2$, $R_3$, $R_4^i$, and $R_5^i$:

- the terms acyl, alkyl, hydroxyalkyl, carboxyalkyl, alkoxyalkyl and alkoxy denote straight or branched chains having 1 to 10 carbon atoms;
- the alkenyl groups consist of straight or branched chains having 2 to 10 carbon atoms;
- the term aryl denotes the phenyl group,

which comprises reacting a compound of the formula VII

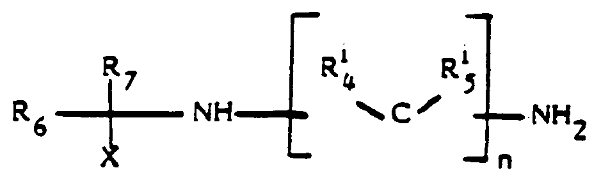

VII

with a dione of the formula VIII

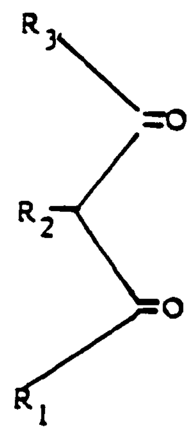

2. The process for the preparation of a compound as claimed in claim 1, wherein X is one of the compounds represented by the formula II or one of the dehydro derivatives thereof:

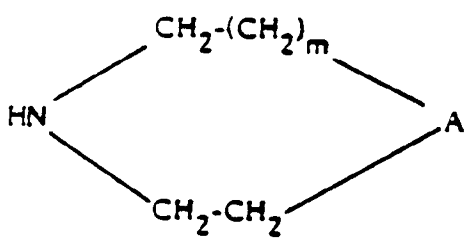

II

in which

| | |
|---|---|
| m | is 0 or 1 and |
| A | is O, N-$R_{11}$ or CH-$R_{11}$, where $R_{11}$ represents H or substituted or unsubstituted alkyl or aryl. |

3. The process for the preparation of a compound as claimed in claim 2, wherein X is a pyrrolyl, imidazolyl, oxazolyl, pyrazolyl, pyridinyl or pyrimidinyl group.

**4.** The process for the preparation of a compound as claimed in one of claims 1 to 3, represented by the formula I, wherein

. X =

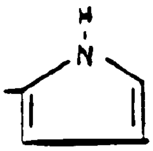 , 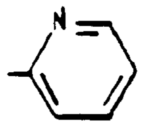 ,

where

. n is 2 or 3,

. $R_4^i = R_5^i = H$ where i = 1 to n or 1 to n-1,

. $R_1$ and $R_3$ independently of one another represent H, $CH_3$, $CF_3$, $C_2H_5$ and $C(CH_3)_3$, and $R_2$ = H.

**5.** The process for the preparation of a compound as claimed in one of claims 1 to 4, which corresponds to the formula III:

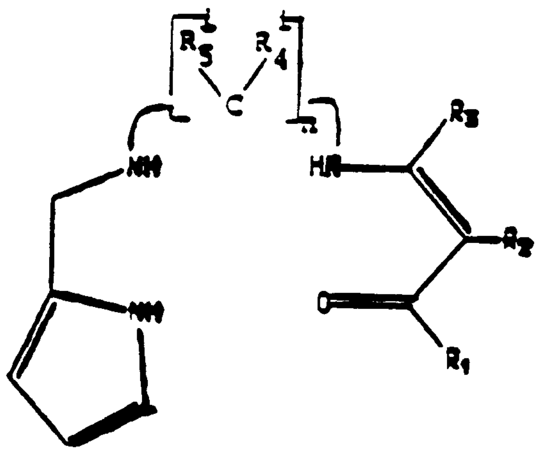

III

in which $R_1$, $R_2$, $R_3$, $R_4^i$ and $R_5^i$ have the meanings given above.

**6.** The process for the preparation of a compound as claimed in one of claims 1 to 4, which corresponds to the formula IV:

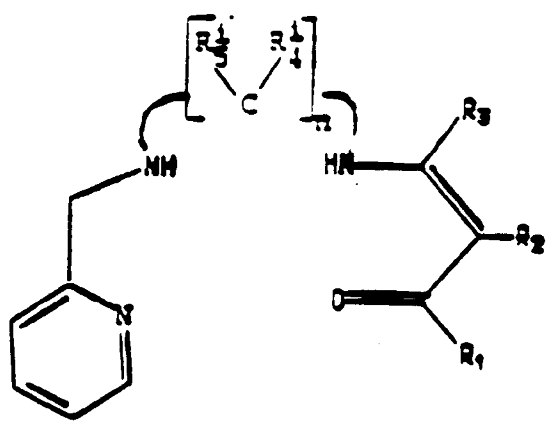

IV

in which $R_1$, $R_2$, $R_3$, $R_4^i$ and $R_5^i$ have the meanings given above in the case of the general formula I.

**7.** The process for the preparation of a compound as claimed in one of claims 1 to 4, which corresponds to the formula VI

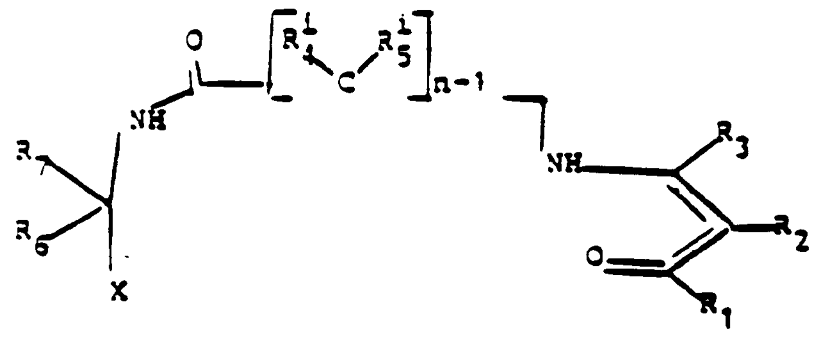

VI

in which $R_1$ to $R_7$ have the meanings given above in the case of general formula I.

8. The process for the preparation of a compound as claimed in claim 7, which corresponds to the formula VI, wherein $R_6 = R_7 = H$, X = heterocycle, $R_4^i = R_5^i = H$, and i = 1 to n-1.

9. The preparation process as claimed in claim 8, wherein the compound of the formula VII is prepared by reacting a heterocycle X which is substituted by a formyl group of the formula

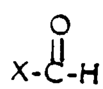

with an amine functional group of an alkylenediamine of the formula $NH_2$-$(CR_4^iR_5^i)_n$-$NH_2$ in acetonitrile in the presence of $NaBH_4$.

10. The process as claimed in claim 9, wherein, when

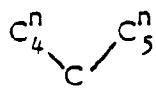 = 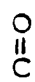 ,

the amine $X(CR_6R_7)NH_2$ is reacted with the amino acid $HOOC(CR_4^iR_5^i)_{n-1}$-$NH_2$ by activating the acid functional group with an activating group customary in peptide chemistry and by protecting the $NH_2$ functional group by a customary protective group.

11. The process for the preparation of a compound as claimed in one of claims 1 to 8, with a metal or a radioactive or paramagnetic metal ion.

12. The process for the preparation of a coordination complex as claimed in claim 11, wherein the metal is $^{111}In$, $^{113m}In$, $^{67}Ga$, $^{68}Ga$, $^{157}Gd$, $^{201}Ti$, $^{117m}Sn$, $^{64}Cu$, $^{186}Re$, $^{188}Re$, $^{90}Y$, $^{212}Bi$, $^{99m}Tc$, $^{97}Ru$, $^{51}Cr$, $^{57}Co$ and $^{191}Os$.

13. The process for the preparation of a coordination complex as claimed in claim 12, wherein the metal is $^{99m}Tc$.

## Patentansprüche

## Patentansprüche für folgende Vertragsstaaten : AT, BE, CH, DE, DK, FR, GB, GR, IT, LI, LU, NL, SE

1. Verbindungen mit der folgenden allgemeinen Formel:

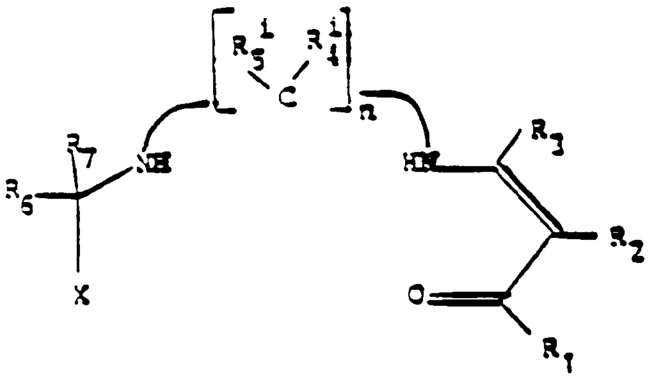

I

worin

| | |
|---|---|
| $R_1$ und $R_3$ | unabhängig voneinander H, eine Alkyl-, Alkenyl-, Cycloalkylgruppe mit 5, 6 oder 7 Kohlenstoffatomen, Aryl-, Arylalkyl-, eine Alylarylgruppe, einen 5- oder 6-gliedrigen Heterocyclus, enthaltend mindestens ein Stickstoffatom, wobei die Gruppen gegebenenfalls substituiert sind, insbesondere durch eine oder mehrere Hydroxy-, Alkoxy- oder Halogengruppe(n), bedeuten; |
| n | eine ganze Zahl von 1 bis 5 bedeutet; |
| i | entsprechend den Werten von 1 bis n für die n aufeinanderfolgenden Kettenglieder |

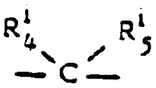

einnimmt,

| | |
|---|---|
| $R_2$, $R^i_4$ und $R^i_5$ | unabhängig voneinander H, eine Alkyl-, Alkenyl-, Aryl-, Alkoxy-, Hydroxyalkyl-, Alkoxyalkyl-, Amid-, Acyl- oder Carboxyalkylgruppe oder ein Salz oder einen Alkylester von letzterem bedeuten; oder $R^i_4$ und $R^i_5$ bilden gemeinsam eine Oxogruppe |

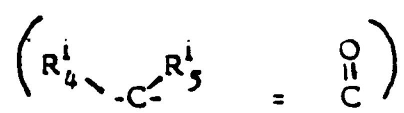

| | |
|---|---|
| $R_6$, $R_7$ | H bedeuten, |
| X | einen 5- oder 6-gliedrigen Heterocyclus, enthaltend wenigstens ein Stickstoffatom bedeutet; |

in $R_1$, $R_2$, $R_3$, $R^i_4$ und $R^i_5$:

- beziehen sich die Ausdrücke Acyl, Alkyl, Hydroxy-, Carboxy- oder Alkoxyalkyl und Alkoxy auf gerade oder verzweigte Ketten mit 1 bis 10 Kohlenstoffatom(en);
- bestehen die Alkenylgruppen aus geraden oder verzweigten Ketten mit 2 bis 10 Kohlenstoffatom(en);
- bezieht sich der Ausdruck Phenyl auf eine Phenylgruppe.

2. Verbindungen nach Anspruch 1, dadurch gekennzeichnet, daß X eine der Verbindungen ist dargestellt durch die Formel II oder eines ihrer Dehydroderivate:

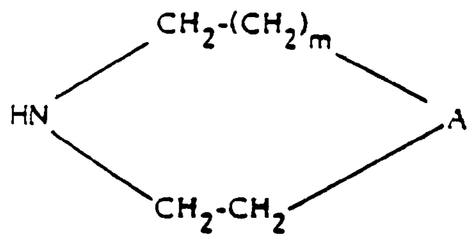 II

worin

m 0 oder 1 ist und

A O, N-$R_{11}$ oder CH-$R_{11}$ ist, worin $R_{11}$ H, gegebenenfalls substituiertes Alkyl oder Aryl bedeutet.

**3.** Verbindungen nach Anspruch 2, dadurch gekennzeichnet, daß X eine Pyrrolyl-, Imidazolyl-, Oxazolyl-, Pyrazolyl-, Pyridinyl-, Pyrimidylgruppe ist.

**4.** Verbindungen nach einem der Ansprüche 1 bis 3, dargestellt durch die Formel I, dadurch gekennzeichnet, daß

- X =

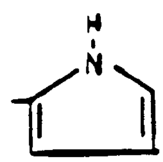 , 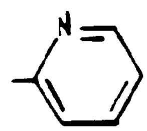

mit

- n = 2 oder 3
- $R^i_4$ = $R^i_5$ = H mit i = 1 bis n oder 1 bis n-1
- $R_1$ und $R_3$ unabhängig voneinander H, $CH_3$, $CF_3$, $C_2H_5$, $C(CH_3)_3$ und $R_2$ = H bedeuten.

**5.** Verbindungen nach einen der Ansprüche 1 bis 4, entsprechend der Formel III:

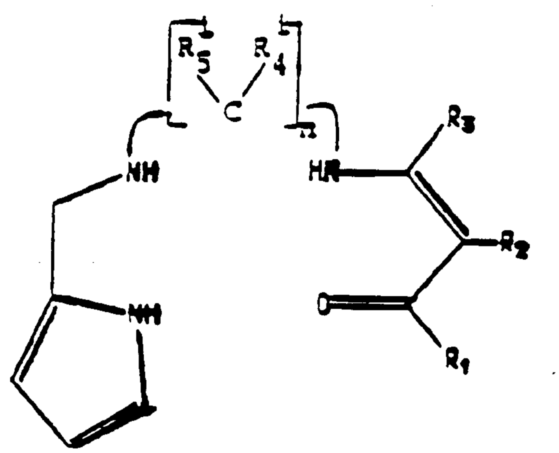 III

worin $R_1$, $R_2$, $R_3$, $R^i_4$ und $R^i_5$ die voranstehend genannten Bedeutungen haben.

**6.** Verbindungen nach einem der Ansprüche 1 bis 4, entsprechend der Formel IV:

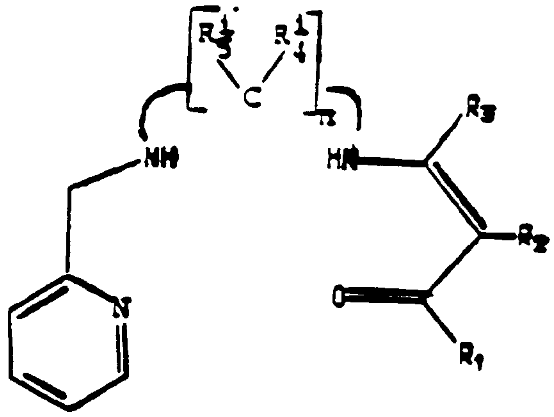

IV

worin $R_1$, $R_2$, $R_3$, $R^i_4$, $R^i_5$ die voranstehend für die allgemeine Formel I genannten Bedeutungen haben.

**7.** Verbindung nach einem der Ansprüche 1 bis 4, entsprechend der formel VI

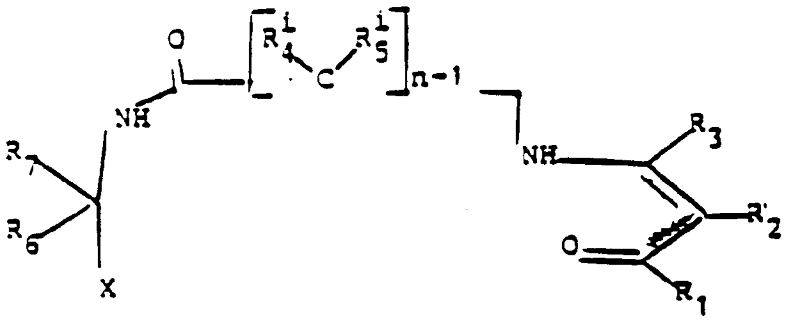

VI

worin $R_1$ bis $R_7$ die voranstehend für die allgemeine Formel I genannten Bedeutungen haben.

**8.** Verbindung nach Anspruch 7, entsprechend der Formel VI, dadurch gekennzeichnet, daß $R_6 = R_7 = H$, X = Heterocyclus, $R^i_4 = R^i_5 = H$, i = 1 bis n-1.

**9.** Verfahren zur Herstellung einer Verbindung nach einem der Ansprüche 1 bis 8, dadurch gekennzeichnet, daß man eine Verbindung mit der Formel VII

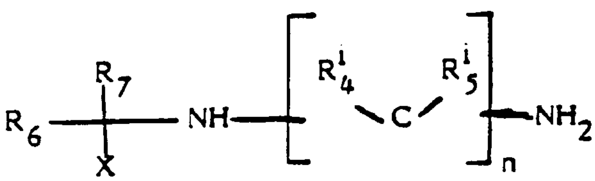

VII

mit einem Dion der Formel VIII umsetzt

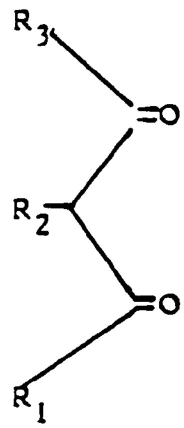

10. Herstellungsverfahren nach Anspruch 9, dadurch gekennzeichnet, daß man die Verbindung mit der Formel VII herstellt, indem man einen Heterocyclus X, substituiert mit einer Formylgruppe der Formel

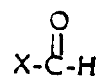

mit einer Aminfunktion eines Alkylendiamins der Formel $NH_2(CR^{i}_4R^{i}_5)-NH_2$ in Acetonitril in Gegenwart von $NaBH_4$ umsetzt.

11. Verfahren nach Anspruch 10, dadurch gekennzeichnet, daß man als

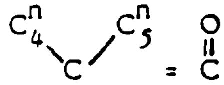

das Amin X $(CR_6C_7)NH_2$ mit Aminosäure $HOOC(CR^{i}_4C^{i}_5)_{n-1}-NH_2$ umsetzt, indem die Säurefunktion durch eine in der Peptidchemie übliche Aktivatorgruppe aktiviert und die $NH_2$-Funktion durch eine übliche Schutzgruppe geschützt wird.

12. Koordinationskomplex einer Verbindung nach einem der Ansprüche 1 bis 8 mit einem radioaktiven oder paramagnetischen Metall oder Metallion.

13. Koordinationskomplex nach Anspruch 12, dadurch gekennzeichnet, daß das Metall $^{111}$In, $^{113m}$In, $^{67}$Ga, $^{68}$Ga, $^{157}$Gd, $^{201}$Ti, $^{117m}$Sn, $^{64}$Cu, $^{186}$Re, $^{188}$Re, $^{90}$Y, $^{212}$Bi, $^{99}$Tc, $^{99m}$Tc, $^{97}$Ru, $^{51}$Cr, $^{57}$Co, $^{191}$Os ist.

14. Koordinationskomplex nach Anspruch 13, dadurch gekennzeichnet, daß das Metall $^{99m}$Tc ist.

15. Kit, enthaltend einen Liganden bestehend aus einer Verbindung nach einem der Ansprüche 1 bis 8, und Reagenzien, die die Bildung von Metallkomplexen nach einem der Ansprüche 12, 13 und 14 erlauben, wenn der Kit in Gegenwart einer der Metallverbindungen erneut gebildet wird.

16. Kit nach dem voranstehenden Anspruch, dadurch gekennzeichnet, daß die Metallverbindung eine Pertechnetatverbindung von $^{99m}$Tc ist, wobei die Reagenzien, die zur Bildung des Koordinationskomplexes führen, ein Reduktionsmittel, wie ein Zinnsalz, enthalten.

17. Kit nach den voranstehenden Ansprüchen, dadurch gekennzeichnet, daß die Verbindung nach den Ansprüchen 1 bis 8 eine wäßrige alkoholische Lösung ist oder lyophilisiert vorliegt.

18. Kit nach Anspruch 15, dadurch gekennzeichnet, daß die Reagenzien auch einen Stabilisator wie para-Aminobenzoesäure oder eine Base enthalten.

19. Kit nach einem der voranstehenden Ansprüche, verwendet zur medizinischen Darstellung des Gehirns, des Herzen, von Infektionen, oder zur Markierung von Lymphocyten zur Darstellung von entzündlichen

Stellen.

**20.** Kit zur Darstellung des Gehirns oder Markierung von weißen Blutkörperchen nach einem der voranstehenden Ansprüche, dadurch gekennzeichnet, daß die zum Wiederherstellen des Kits verwendete metallische Verbindung das Pertechnetat $^{99m}TcO^{-}_{4}$ ist.

**Patentansprüche für folgenden Vertragsstaat : ES**

**1.** Verfahren zur Herstellung von Verbindungen mit der folgenden allgemeinen Formel:

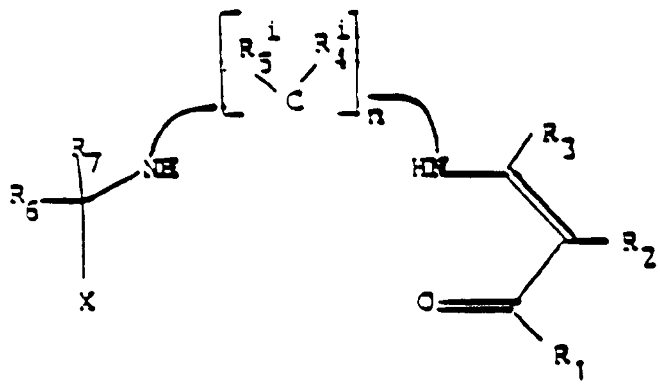

I

worin

$R_1$ und $R_3$ unabhängig voneinander H, eine Alkyl-, Alkenyl-, Cycloalkylgruppe mit 5, 6 oder 7 Kohlenstoffatomen, Aryl-, Arylalkyl-, eine Alylarylgruppe, einen 5- oder 6-gliedrigen Heterocyclus, enthaltend mindestens ein Stickstoffatom, wobei die Gruppen gegebenenfalls substituiert sind, insbesondere durch eine oder mehrere Hydroxy-, Alkoxy- oder Halogengruppe(n), bedeuten;

n eine ganze Zahl von 1 bis 5 bedeutet;

i entsprechend den Werten von 1 bis n für die n aufeinanderfolgenden Kettenglieder

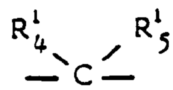

einnimmt,

$R_2$, $R^i_4$ und $R^i_5$ unabhängig voneinander H, eine Alkyl-, Alkenyl-, Aryl-, Alkoxy-, Hydroxyalkyl-, Alkoxyalkyl-, Amid-, Acyl- oder Carboxyalkylgruppe oder ein Salz oder einen Alkylester von letzterem bedeuten; oder $R^i_4$ und $R^i_5$ bilden gemeinsam eine Oxogruppe

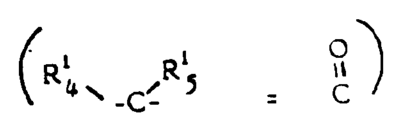

$R_6$, $R_7$ H bedeuten,

X einen 5- oder 6-gliedrigen Heterocyclus, enthaltend wenigstens ein Stickstoffatom bedeutet;

in $R_1$, $R_2$, $R_3$, $R^i_4$ und $R^i_5$:

- beziehen sich die Ausdrücke Acyl, Alkyl, Hydroxy-, Carboxy- oder Alkoxyalkyl und Alkoxy auf gerade oder verzweigte Ketten mit 1 bis 10 Kohlenstoffatom(en);
- bestehen die Alkenylgruppen aus geraden oder verzweigten Ketten mit 2 bis 10 Kohlenstoffatom(en);
- bezieht sich der Ausdruck Phenyl auf eine Phenylgruppe,

dadurch gekennzeichnet, daß man eine Verbindung mit der Formel VII

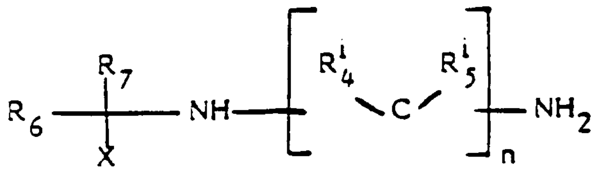

VII

mit einem Dion der Formel VIII umsetzt

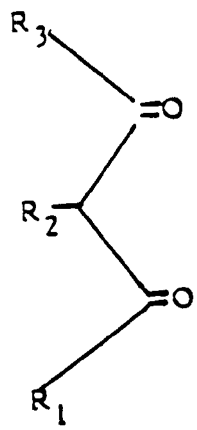

2. Verfahren zur Herstellung von Verbindungen nach Anspruch 1, dadurch gekennzeichnet, daß X eine der Verbindungen ist dargestellt durch die Formel II oder eines ihrer Dehydroderivate:

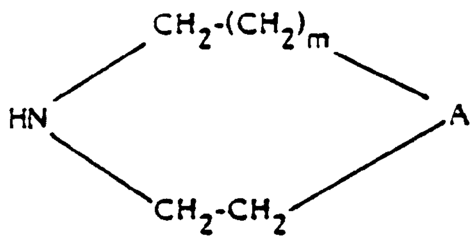

II

worin

m 0 oder 1 ist und

A O, N-$R_{11}$ oder CH-$R_{11}$ ist, worin $R_{11}$ H, gegebenenfalls substituiertes Alkyl oder Aryl bedeutet.

3. Verfahren zur Herstellung von Verbindungen nach Anspruch 2, dadurch gekennzeichnet, daß X eine Pyrrolyl-, Imidazolyl-, Oxazolyl-, Pyrazolyl-, Pyridinyl-, Pyrimidylgruppe ist.

4. Verfahren zur Herstellung von Verbindungen nach einem der Ansprüche 1 bis 3, dargestellt durch die Formel I, dadurch gekennzeichnet, daß

- X =

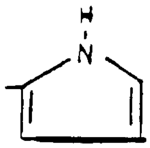

,

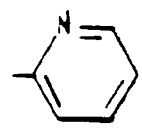

mit

- n = 2 oder 3
- $R^i_4 = R^i_5$ = H mit i = 1 bis n oder 1 bis n-1

- $R_1$ und $R_3$ unabhängig voneinander H, $CH_3$, $CF_3$, $C_2H_5$, $C(CH_3)_3$ und $R_2$ = H bedeuten.

5. Verfahren zur Herstellung von Verbindungen nach einem der Ansprüche 1 bis 4, entsprechend der Formel III:

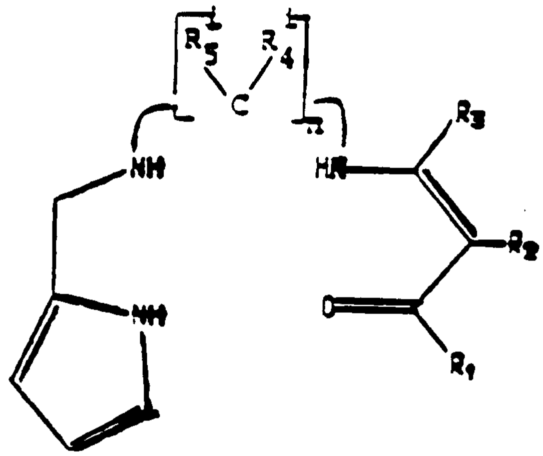

III

worin $R_1$, $R_2$, $R_3$, $R^i_4$ und $R^i_5$ die voranstehend genannten Bedeutungen haben.

6. Verfahren zur Herstellung von Verbindungen nach einem der Ansprüche 1 bis 4, entsprechend der Formel IV:

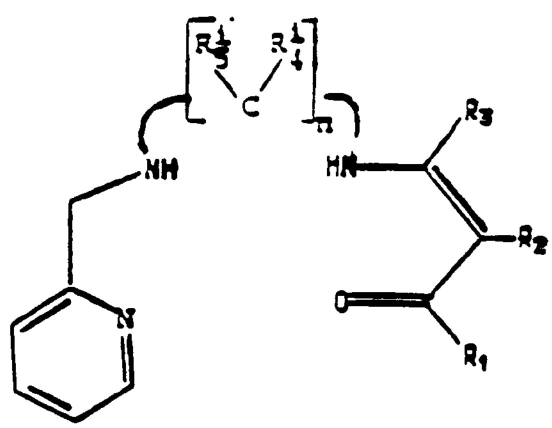

IV

worin $R_1$, $R_2$, $R_3$, $R^i_4$, $R^i_5$ die voranstehend für die allgemeine Formel I genannten Bedeutungen haben.

7. Verfahren zur Herstellung einer Verbindung nach einem der Ansprüche 1 bis 4, entsprechend der Formel VI

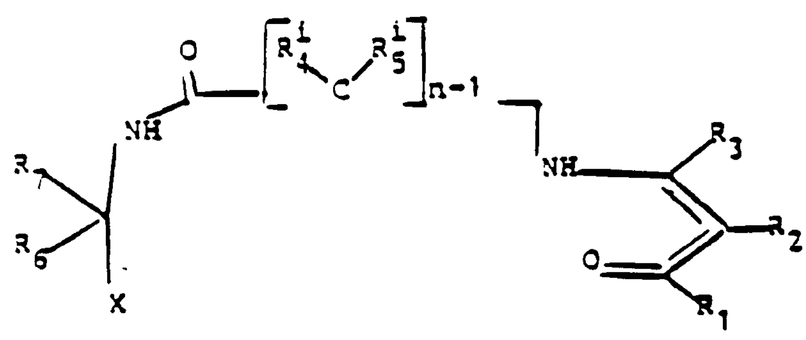

VI

worin $R_1$ bis $R_7$ die voranstehend für die allgemeine Formel I genannten Bedeutungen haben.

8. Verfahren zur Herstellung einer Verbindung nach Anspruch 7, entsprechend der Formel VI, dadurch gekennzeichnet, daß $R_6 = R_7 = H$, X = Heterocyclus, $R^i_4 = R^i_5 = H$, i = 1 bis n-1.

9. Herstellungsverfahren nach Anspruch 8, dadurch gekennzeichnet, daß man die Verbindung mit der Formel VII herstellt, indem man einen Heterocyclus X, substituiert mit einer Formylgruppe der Formel

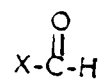

mit einer Aminfunktion eines Alkylendiamins der Formel $NH_2(CR^i_4R^i_5)$-$NH_2$ in Acetonitril in Gegenwart von $NaBH_4$ umsetzt.

10. Verfahren nach Anspruch 10, dadurch gekennzeichnet, daß man als

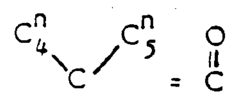

das Amin X $(CR_6C_7)NH_2$ mit Aminosäure $HOOC(CR^i_4C^i_5)_{n-1}$-$NH_2$ umsetzt, indem die Säurefunktion durch eine in der Peptidchemie übliche Aktivatorgruppe aktiviert und die $NH_2$-Funktion durch eine übliche Schutzgruppe geschützt wird.

11. Verfahren zur Herstellung einer Verbindung nach einem der Ansprüche 1 bis 8, mit einem radioaktiven oder paramagnetischen Metall oder Metallion.

12. Verfahren zur Herstellung einer Verbindung nach einem der Ansprüche 1 bis 8, dadurch gekennzeichnet, daß das Metall $^{111}In$, $^{113m}In$, $^{67}Ga$, $^{68}Ga$, $^{157}Gd$, $^{201}Ti$, $^{117m}Sn$, $^{64}Cu$, $^{186}Re$, $^{188}Re$, $^{90}Y$, $^{212}Bi$, $^{99}Tc$, $^{99m}Tc$, $^{97}Ru$, $^{51}Cr$, $^{57}Co$, $^{191}Os$ ist.

13. Verfahren zur Herstellung einer Verbindung nach Anspruch 12, dadurch gekennzeichnet, daß das Metall $^{99m}Tc$ ist.

FIGURE 1

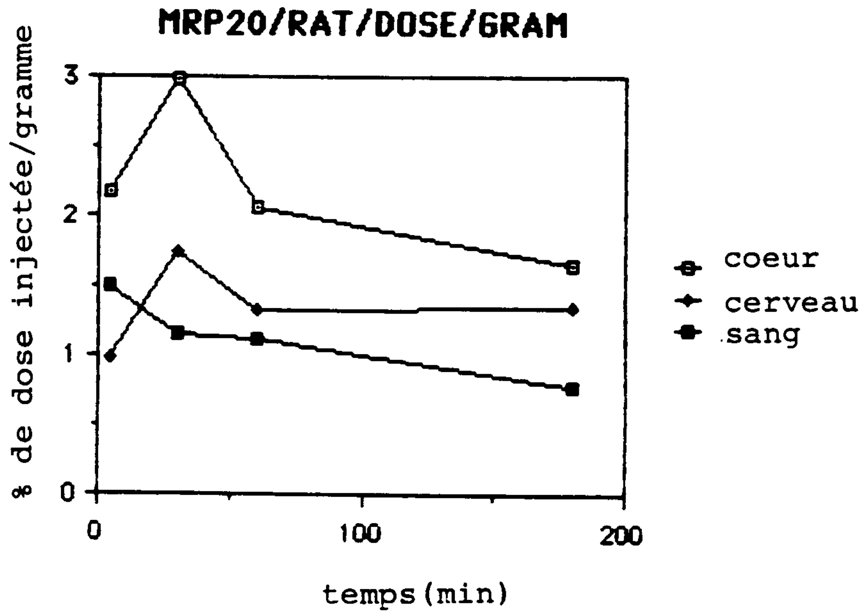
MRP20/RAT/DOSE/GRAM
% de dose injectée/gramme
3
2
1
0
0
100
200
temps(min)
coeur
cerveau
sang

FIGURE 2

MRP30 BIODISTRIBUTION/RATS

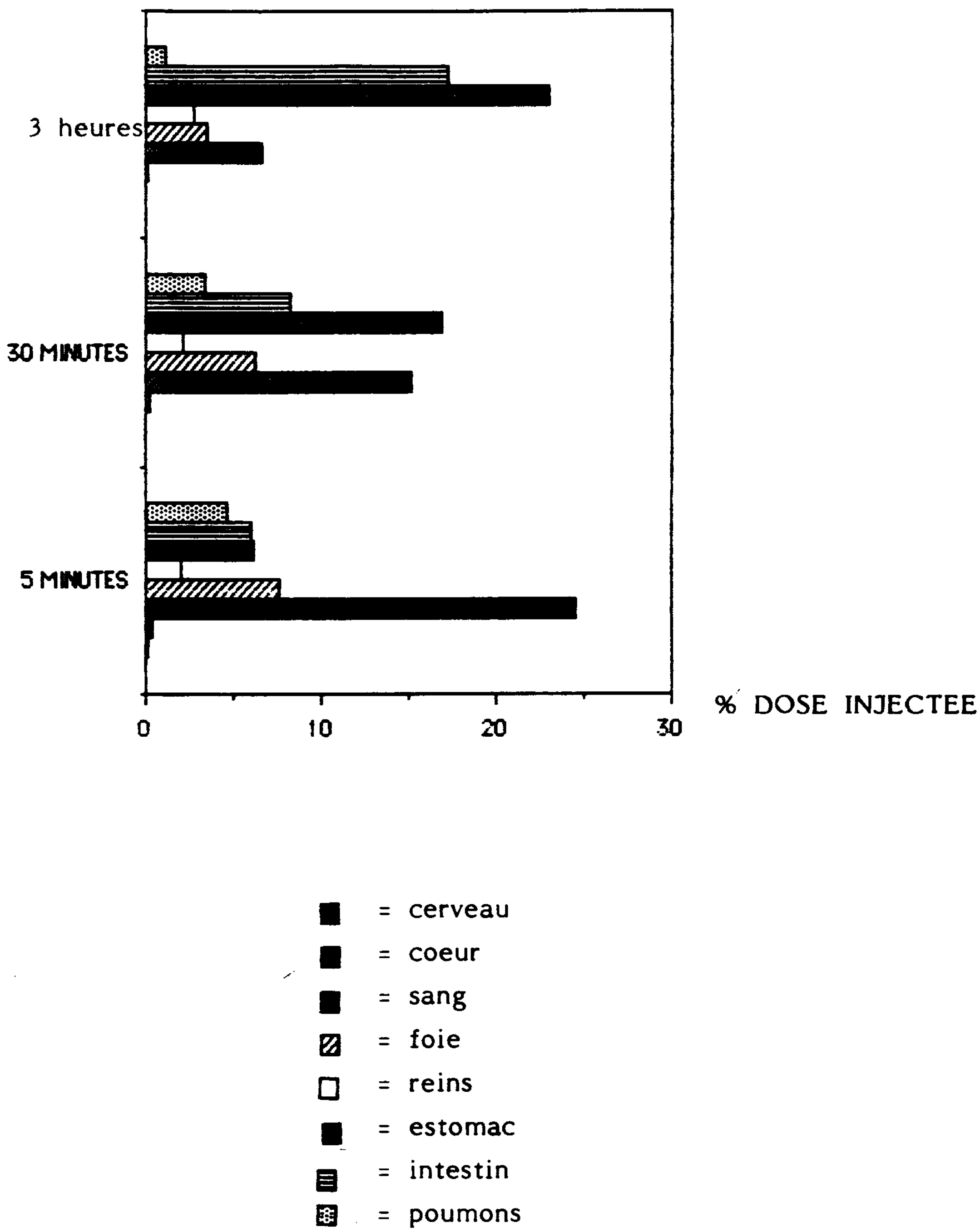

FIGURE 3

MRP27:% DOSE/ORGANE RAT

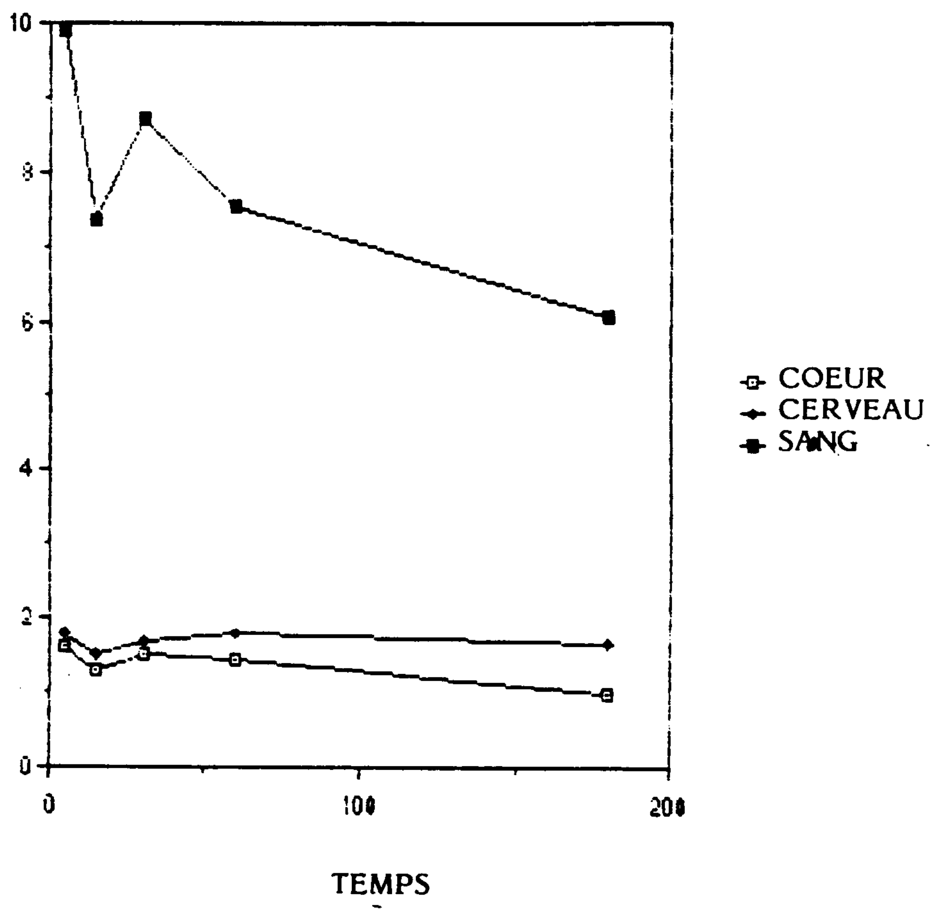

FIGURE 4

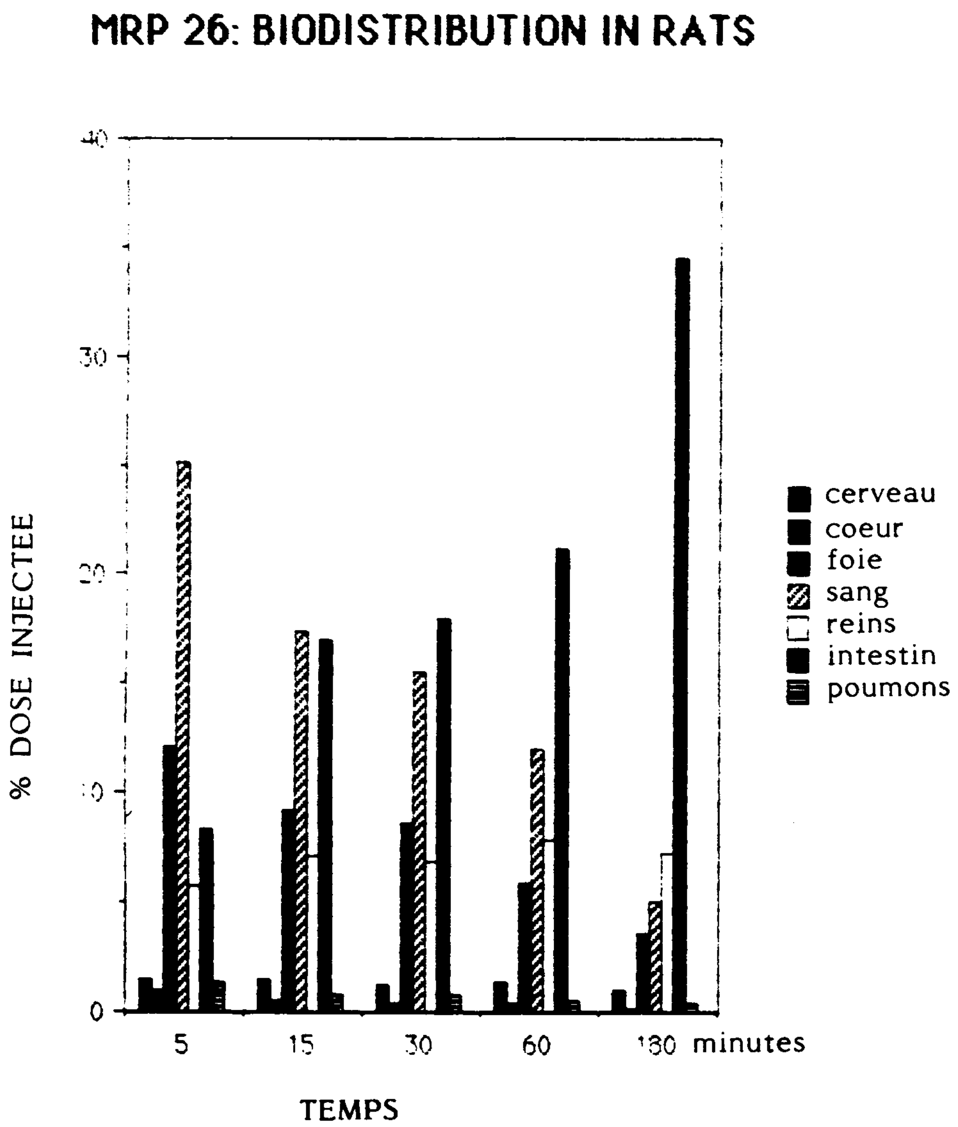
MRP 26: BIODISTRIBUTION IN RATS
% DOSE INJECTEE
40
30
20
10
0
5
15
30
60
180 minutes
TEMPS
cerveau
coeur
foie
sang
reins
intestin
poumons